(19) Europäisches Patentamt
European Patent Office
Office européen des brevets

(11) EP 1 472 278 B1

(12) **EUROPEAN PATENT SPECIFICATION**

(45) Date of publication and mention of the grant of the patent:
**12.11.2008 Bulletin 2008/46**

(21) Application number: **03700743.2**

(22) Date of filing: **24.01.2003**

(51) Int Cl.:
***C07K 5/08*** (2006.01) ***A61K 38/06*** (2006.01)
***A61P 31/14*** (2006.01)

(86) International application number:
**PCT/CA2003/000089**

(87) International publication number:
**WO 2003/064455 (07.08.2003 Gazette 2003/32)**

(54) **MACROCYCLIC PEPTIDES ACTIVE AGAINST THE HEPATITIS C VIRUS**

MAKROZYKLISCHE PEPTIDE MIT ANTI-HEPATITIS C VIRUS WIRKUNG

PEPTIDES MACROCYCLIQUES ACTIFS CONTRE LE VIRUS DE L'HEPATITE C

(84) Designated Contracting States:
**AT BE BG CH CY CZ DE DK EE ES FI FR GB GR HU IE IT LI LU MC NL PT SE SI SK TR**
Designated Extension States:
**LT LV RO**

(30) Priority: **30.01.2002 CA 2369711**

(43) Date of publication of application:
**03.11.2004 Bulletin 2004/45**

(73) Proprietor: **BOEHRINGER INGELHEIM (CANADA) LTD.**
**Laval, Québec, H7S 2G5 (CA)**

(72) Inventors:
- **LLINAS-BRUNET, Montse**
  **Laval, Québec H7S 2G5 (CA)**
- **GORYS, Vida, J.**
  **Laval, Québec H7S 2G5 (CA)**

(74) Representative: **Mahlbacher, Volker et al**
**Boehringer Ingelheim GmbH,**
**CD Patents**
**55216 Ingelheim (DE)**

(56) References cited:
**WO-A-00/59929**
**WO-A-03/053349**
**US-B1- 6 608 027**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 472 278 B1

**SEQUENCE LISTING**

<110> BOEHRINGER INGELHEIM (CANADA) LTD.

<120> MARCROCYCLIC PEPTIDES ACTIVE AGAINST THE HEPATITIS C VIRUS

<130> 13/092

<150> 2,369,711
<151> 2002-01-30

<160> 3

<170> FastSEQ for Windows Version 4.0

<210> 1
<211> 30
<212> DNA
<213> Artificial Sequence

<220>
<223> Forward primer

<400> 1
acgcagaaag cgtctagcca tggcgttagt 30

<210> 2
<211> 30
<212> DNA
<213> Artificial Sequence

<220>
<223> Reverse primer

<400> 2
tcccggggca ctcgcaagca ccctatcagg 30

<210> 3
<211> 26
<212> DNA
<213> Artificial Sequence

<220>
<223> PUTR probe

<400> 3
tggtctgcgg aaccggtgag tacacc 26

## Description

### FIELD OF THE INVENTION

The present invention relates to compounds, processes for their synthesis, compositions and uses of said compounds for the manufacture of a medicament for the treatment of hepatitis C virus (HCV) infection. In particular, the present invention provides novel peptide analogs, pharmaceutical compositions containing such analogs and the use of these analog for the manufacture of a medicament for the treatment of HCV infection.

### BACKGROUND OF THE INVENTION

Hepatitis C virus (HCV) is the major etiological agent of post-transfusion and community-acquired non-A non-B hepatitis worldwide. It is estimated that over 200 million people worldwide are infected by the virus. A high percentage of carriers become chronically infected and many progress to chronic liver disease, so-called chronic hepatitis C. This group is in turn at high risk for serious liver disease such as liver cirrhosis, hepatocellular carcinoma and terminal liver disease leading to death.

The mechanism by which HCV establishes viral persistence and causes a high rate of chronic liver disease has not been thoroughly elucidated. It is not known how HCV interacts with and evades the host immune system. In addition, the roles of cellular and humoral immune responses in protection against HCV infection and disease have yet to be established. Immunoglobulins have been reported for prophylaxis of transfusion-associated viral hepatitis, however, the Center for Disease Control does not presently recommend immunoglobulins treatment for this purpose. The lack of an effective protective immune response is hampering the development of a vaccine or adequate post-exposure prophylaxis measures, so in the near-term, hopes are firmly pinned on antiviral interventions.

Various clinical studies have been conducted with the goal of identifying pharmaceutical agents capable of effectively treating HCV infection in patients afflicted with chronic hepatitis C. These studies have involved the use of interferon-alpha, alone and in combination with other antiviral agents. Such studies have shown that a substantial number of the participants do not respond to these therapies, and of those that do respond favorably, a large proportion were found to relapse after termination of treatment.

Until recently, interferon (IFN) was the only available therapy of proven benefit approved in the clinic for patients with chronic hepatitis C. However the sustained response rate is low, and interferon treatment also induces severe side-effects (i.e. retinopathy, thyroiditis, acute pancreatitis, depression) that diminish the quality of life of treated patients. Recently, interferon in combination with ribavirin has been approved for patients non-responsive to IFN alone. However, the side effects caused by IFN are not alleviated with this combination therapy. Pegylated forms of interferons such as PEG-Intron® and Pegasys® can apparently partially address these deleterious side-effects but antiviral drugs still remain the avenue of choice for oral treatment of HCV.

Therefore, a need exists for the development of effective antiviral agents for treatment of HCV infection that overcome the limitations of existing pharmaceutical therapies.

HCV is an enveloped positive strand RNA virus in the Flaviviridae family. The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature nonstructural proteins (NS2, NS3, NS4A, NS4B, NS5A, and NS5B) is effected by two viral proteases. The first one, as yet poorly characterized, cleaves at the NS2-NS3 junction (henceforth referred to as NS2/3 protease); the second one is a serine protease contained within the N-terminal region of NS3 (NS3 protease) and mediates all the subsequent cleavages downstream of NS3, both in *cis,* at the NS3-NS4A cleavage site, and in *trans,* for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protease with NS4A seems necessary to the processing events, enhancing the proteolytic efficiency at all of the sites. The NS3 protein also exhibits nucleoside triphosphatase and RNA helicase activities. NS5B is a RNA-dependent RNA polymerase that is involved in the replication of HCV.

A general strategy for the development of antiviral agents is to inactivate virally encoded enzymes that are essential for the replication of the virus.

The following is a list of patent applications published in the last few years that disclose HCV NS3 protease inhibitor peptide analogs that are structurally different from the compounds of the present invention:

GB 2,337,262; JP10298151; JP 11126861; JP 11292840; JP 2001-103993;
US 6,159,938; US 6,187,905; WO 97/43310; WO 98/17679; WO 98/22496;
WO 98/46597; WO 98/46630; WO 99/38888; WO 99/50230; WO 99/64442;

WO 99/07733; WO 99/07734; WO 00/09543; WO 00/09558; WO 00/20400; WO 00/59929; WO 00/31129; WO 01/02424; WO 01/07407; WO 01 /16357; WO 01/32691; WO 01/40262; WO 01/58929; WO 01/64678; WO 01/74768; WO 01/77113; WO 01/81325; WO 02/08187; WO 02/08198; WO 02/08244; WO 02/08251; WO 02/08256; WO 02/18369; WO 02/60926 and WO 02/79234.

The compounds of the present invention distinguish themselves by having a different chemical structure and by the surprising finding that they specifically inhibit HCV NS3 protease while showing insignificant inhibitory activity against other serine proteases. Furthermore, the compounds are active in cell culture and have surprisingly good pharmacokinetic profile *in vivo.*

## SUMMARY OF THE INVENTION

Included in the scope of the invention are compounds of formula I:

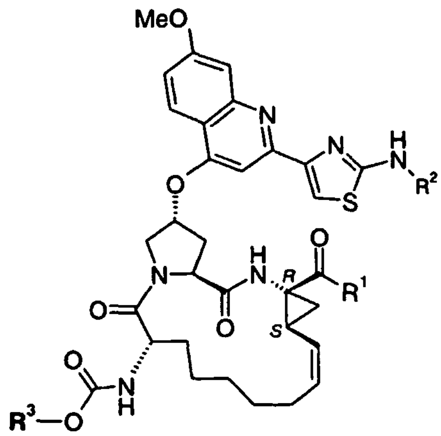

**(I)**

wherein $\mathbf{R^1}$ is hydroxy or $NHSO_2\mathbf{R^{1A}}$ wherein $\mathbf{R^{1A}}$ is $(C_{1-8})$alkyl, $(C_{3-7})$cycloalkyl or {$(C_{1-6})$alkyl-$(C_{3-7})$cycloalkyl}, which are all optionally substituted from 1 to 3 times with halo, cyano, nitro, O-$(C_{1-6})$alkyl, amido, amino or phenyl, or $\mathbf{R^{1A}}$ is $C_6$ or $C_{10}$ aryl which is optionally substituted from 1 to 3 times with halo, cyano, nitro, $(C_{1-6})$alkyl, O-$(C_{1-6})$alkyl, amido, amino or phenyl: $\mathbf{R^2}$ is $(C_{5-6})$cycloalkyl and $\mathbf{R^3}$ is cyclopentyl; or a pharmaceutically acceptable salt thereof.

Included within the scope of this invention is a pharmaceutical composition comprising an anti-hepatitis C virally effective amount of a compound of formula I, or a therapeutically acceptable salt thereof, in admixture with a pharmaceutically acceptable carrier medium or auxiliary agent.

According to one embodiment, the pharmaceutical composition of this invention further interferon (pegylated or not), or ribavirin, or one or more other anti-HCV agent, or any combination of the above.

The invention allows for a compound of formula I, a therapeutically acceptable salt thereof, or a composition as described above, alone or in combination with one ore more of: interferon (pegylated or not), or ribavirin, or one or more other anti-HCV agent, all of which administered together or separately, for use in the treatment of a hepatitis C viral infection in a mammal, by administration of an anti-hepatitis C virally effective compound.

Additionally, the invention allows for a compound of formula I, a therapeutically acceptable salt thereof, or a composition as described above, alone or in combination with one ore more of: interferon (pegylated or not), or ribavirin, or one or more other anti-HCV agent, all of which administered together or separately, for the prevention of a hepatitis C viral infection in a mammal, by administration of an anti-hepatitis C virally effective compound.

Also within the scope of this invention is the use of a compound of formula I, as described herein, for the manufacture of a medicament for the treatment or prevention of hepatitis C viral infection.

## DETAILED DESCRIPTION OF PREFERRED EMBODIMENTS

### Definitions

As used herein, the following definitions apply unless otherwise noted:

With reference to the instances where *(R)* or *(S)* is used to designate the absolute configuration of an asymmetric center, the designation is done in the context of the whole compound and not in the context of the substituent alone.

The designation "P1, P2, and P3" as used herein refer to the position of the amino acid residues starting from the C-terminus end of the peptide analogs and extending towards the N-terminus (i.e. P1 refers to position 1 from the C-terminus, P2: second position from the C-terminus, etc.) (see Berger A. & Schechter I., Transactions of the Royal Society London series B257, 249-264 (1970)).

As used herein the term "*(1R, 2S)*-vinyl-ACCA" refers to a compound of formula:

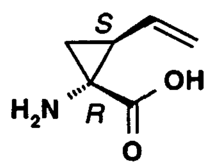

namely, (*1R, 2S*) 1-amino-2-ethenylcyclopropylcarboxylic acid.

The term "halo" as used herein means a halogen substituent selected from bromo, chloro, fluoro or iodo.

The term "($C_{1-6}$)alkyl" or "(lower)alkyl" as used herein, either alone or in combination with another substituent, means acyclic, straight or branched chain alkyl substituents containing from 1 to 6 carbon atoms and includes, for example, methyl, ethyl, propyl, butyl, hexyl, 1-methylethyl, 1-methylpropyl, 2-methylpropyl and 1,1-dimethylethyl.

The term "($C_{1-8}$)alkyl" as used herein, either alone or in combination with another substituent, means acyclic, straight or branched chain alkyl substituents containing 1 to 8 carbon atoms and includes, for example, methyl, ethyl, 2,2-dimethylbutyl, hexyl, 1-methylhexyl, heptyl and octyl.

The term "($C_{3-7}$)cycloalkyl" as used herein, either alone or in combination with another substituent, means a cycloalkyl substituent containing from three to seven carbon atoms and includes cyclopropyl, cyclobutyl, cyclopentyl, cyclohexyl and cycloheptyl.

The term "{($C_{1-6}$)alkyl-($C_{3-7}$)cycloalkyl}" as used herein means a cycloalkyl radical containing from 3 to 7 carbon atoms directly linked to an alkylene radical containing 1 to 6 carbon atoms; for example, cyclopropylmethyl, cyclopentylethyl, cyclohexylmethyl, cyclohexylethyl, and cycloheptylpropyl. In the instance where $\mathbf{R^{3A}}$ is a {($C_{1-6}$)alkyl-($C_{3-7}$)cycloalkyl}, this group is attached to the $SO_2$ group via the ($C_{1-6}$)alkyl (i.e. the alkylene portion).

The term "O-($C_{1-6}$)alkyl" as used herein, either alone or in combination with another substituent, means the substituent -O-($C_{1-6}$)alkyl wherein alkyl is as defined above containing up to six carbon atoms. O-($C_{1-6}$)alkyl includes methoxy, ethoxy, propoxy, 1-methylethoxy, butoxy and 1,1-dimethylethoxy. The latter substituent is known commonly as *tert*-butoxy.

The term "pharmaceutically acceptable salt" means a salt of a compound of formula I which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. Lists of suitable salts are found in, e.g., S.M. Birge et al., J. Pharm. Sci., 1977, 66, pp. 1-19, which is hereby incorporated by reference in its entirety.

The term "pharmaceutically-acceptable acid addition salt" means those salts which retain the biological effectiveness and properties of the free bases and which are not biologically or otherwise undesirable, formed with inorganic acids such as hydrochloric acid, hydrobromic acid, hydroiodic acid, sulfuric acid, sulfamic acid, nitric acid, phosphoric acid, and the like, and organic acids such as acetic acid, trichloroacetic acid, trifluoroacetic acid, adipic acid, alginic acid, ascorbic acid, aspartic acid, benzenesulfonic acid, benzoic acid, 2-acetoxybenzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, heptanoic acid, hexanoic acid, formic acid, fumaric acid, 2-hydroxyethanesulfonic acid (isethionic acid), lactic acid, maleic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, picric acid, pivalic acid, propionic acid, pyruvic acid, salicylic acid, stearic acid, succinic acid, sulfanilic acid, tartaric acid, p-toluenesulfonic acid, undecanoic acid, and the like.

The term "pharmaceutically-acceptable base addition salt" means those salts which retain the biological effectiveness and properties of the free acids and which are not biologically or otherwise undesirable, formed with inorganic bases such as ammonia or hydroxide, carbonate, or bicarbonate of ammonium or a metal cation such as sodium, potassium, lithium, calcium, magnesium, iron, zinc, copper, manganese, aluminum, and the like. Particularly preferred are the ammonium, potassium, sodium, calcium, and magnesium salts. Salts derived from pharmaceutically-acceptable organic nontoxic bases include salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine,

tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, N-ethylpiperidine, tetramethylammonium compounds, tetraethylammonium compounds, pyridine, N,N-dimethylaniline, N-methylpiperidine, N-methylmorpholine, dicyclohexylamine, dibenzylamine, N,N-dibenzylphenethylamine, 1-ephenamine, N,N'-dibenzylethylenediamine, polyamine resins, and the like. Particularly preferred organic nontoxic bases are isopropylamine, diethylamine, ethanolamine, trimethylamine, dicyclohexylamine, choline, and caffeine.

The term "antiviral agent" as used herein means an agent (compound or biological) that is effective to inhibit the formation and/or replication of a virus in a mammal. This includes agents that interfere with either host or viral mechanisms necessary for the formation and/or replication of a virus in a mammal. Antiviral agents include, for example, ribavirin, amantadine, VX-497 (merimepodib, Vertex Pharmaceuticals), VX-498 (Vertex Pharmaceuticals), Levovirin, Viramidine, Ceplene (maxamine), XTL-001 and XTL-002 (XTL Biopharmaceuticals).

The term other anti-HCV agent as used herein means those agents that are effective for diminishing or preventing the progression of hepatitis C related symptoms of disease. Such agents can be selected from: immunomodulatory agents, inhibitors of HCV NS3 protease, inhibitors of HCV polymerase or inhibitors of another target in the HCV life cycle.

The term "immunomodulatory agent" as used herein means those agents (compounds or biologicals) that are effective to enhance or potentiate the immune system response in a mammal. Immunomodulatory agents include, for example, class I interferons (such as α-, β-, δ- and omega interferons, tau-interferons, consensus interferons and asialo-interferons), class II interferons (such as γ-interferons) and pegylated interferons.

The term "inhibitor of HCV NS3 protease" as used herein means an agent (compound or biological) that is effective to inhibit the function of HCV NS3 protease in a mammal. Inhibitors of HCV NS3 protease include, for example, those compounds described in WO 99/07733, WO 99/07734, WO 00/09558, WO 00/09543, WO 00/59929 or WO 02/060926, the Boehringer Ingelheim clinical candidate identified as BILN 2061 and the Vertex/Eli Lilly pre-development candidate identified as VX-950 or LY-570310. Particularly, compounds # 2, 3, 5, 6, 8, 10, 11, 18, 19, 29, 30, 31, 32, 33, 37, 38, 55, 59, 71, 91, 103, 104, 105, 112, 113, 114, 115, 116, 120, 122, 123, 124, 125, 126 and 127 disclosed in the table of pages 224-226 in WO 02/060926, can be used in combination with the compounds of the present invention.

The term "inhibitor of HCV polymerase" as used herein means an agent (compound or biological) that is effective to inhibit the function of an HCV polymerase in a mammal. This includes, for example, inhibitors of HCV NS5B polymerase. Inhibitors of HCV polymerase include non-nucleosides, for example, those compounds described in:

- US Application No. 10/198,680, which corresponds to PCT/CA02/01127, both filed 18 July 2002 (Boehringer Ingelheim),
- US Application No. 10/198,384, which corresponds to PCT/CA02/01128, both filed 18 July 2002 (Boehringer Ingelheim),
- US Application No. 10/198,259, which corresponds to PCT/CA02/01129, both filed 18 July 2002 (Boehringer Ingelheim),
- WO 02/100846 A1 and WO 02/100851 A2 (both Shire),
- WO 01/85172 A1 and WO 02/098424 A1 (both GSK),
- WO 00/06529 and WO 02/06246 A1 (both Merck),
- WO 01/47883 and WO 03/000254 (both Japan Tobacco) and
- EP 1 256 628 A2 (Agouron).

Furthermore other inhibitors of HCV polymerase also include nucleoside analogs, for example, those compounds described in:

- WO 01/90121 A2 (Idenix),
- WO 02/069903 A2 (Biocryst Pharmaceuticals Inc.), and
- WO 02/057287 A2 and WO 02/057425 A2 (both Merck/Isis).

Specific examples of inhibitors of an HCV polymerase, include JTK-002, JTK-003 and JTK-109 (Japan Tobacco).

The term "inhibitor of another target in the HCV life cycle" as used herein means an agent (compound or biological) that is effective to inhibit the formation and/or replication of HCV in a mammal other than by inhibiting the function of the HCV NS3 protease. This includes agents that interfere with either host or HCV viral mechanisms necessary for the formation and/or replication of HCV in a mammal. Inhibitors of another target in the HCV life cycle include, for example, agents that inhibit a target selected from a helicase, an HCV NS2/3 protease and an internal ribosome entry site (IRES). A specific example of inhibitors of another target in the HCV life cycle include ISIS-14803 (ISIS Pharmaceuticals).

The term "HIV inhibitor" as used herein means an agent (compound or biological) that is effective to inhibit the formation and/or replication of HIV in a mammal. This includes agents that interfere with either host or viral mechanisms necessary for the formation and/or replication of HIV in a mammal. HIV inhibitors include, for example, nucleosidic inhibitors, non-nucleosidic inhibitors, protease inhibitors, fusion inhibitors and integrase inhibitors.
The term "HAV inhibitor" as used herein means an agent (compound or biological) that is effective to inhibit the formation and/or replication of HAV in a mammal. This includes agents that interfere with either host or viral mechanisms necessary for the formation and/or replication of HAV in a mammal. HAV inhibitors include Hepatitis A vaccines, for example, Havrix® (GlaxoSmithKline), VAQTA® (Merck) and Avaxim® (Aventis Pasteur).
The term "HBV inhibitor" as used herein means an agent (compound or biological) that is effective to inhibit the formation and/or replication of HBV in a mammal. This includes agents that interfere with either host or viral mechanisms necessary for the formation and/or replication of HBV in a mammal. HBV inhibitors include, for example, agents that inhibit HBV viral DNA polymerase or HBV vaccines. Specific examples of HBV inhibitors include Lamivudine (Epivir-HBV®), Adefovir Dipivoxil, Entecavir, FTC (Coviracil®), DAPD (DXG), L-FMAU (Clevudine®), AM365 (Amrad), Ldt (Telbivudine), monoval-LdC (Valtorcitabine), ACH-126,443 (L-Fd4C) (Achillion), MCC478 (Eli Lilly), Racivir (RCV), Fluoro-L and D nucleosides, Robustaflavone, ICN 2001-3 (ICN), Bam 205 (Novelos), XTL-001 (XTL), Imino-Sugars (Nonyl-DNJ) (Synergy), HepBzyme; and immunomodulator products such as: interferon alpha 2b, HE2000 (Hollis-Eden), Theradigm (Epimmune), EHT899 (Enzo Biochem), Thymosin alpha-1 (Zadaxin®), HBV DNA vaccine (PowderJect), HBV DNA vaccine (Jefferon Center), HBV antigen (OraGen), BayHep B® (Bayer), Nabi-HB® (Nabi) and Anti-hepatitis B (Cangene); and HBV vaccine products such as the following: Engerix B, Recombivax HB, GenHevac B, Hepacare, Bio-Hep B, TwinRix, Comvax, Hexavac.
The term "class I interferon" as used herein means an interferon selected from a group of interferons that all bind to receptor type I. This includes both naturally and synthetically produced class I interferons. Examples of class I interferons include α-, β-, omega interferons, tau-interferons, consensus interferons, asialo-interferons.
The term "class II interferon" as used herein means an interferon selected from a group of interferons that all bind to receptor type II. Examples of class II interferons include γ-interferons.
The pharmaceutical compositions of the invention may contain one or more additional active agents selected, for example, from antiviral agents, immunomodulatory agents, other inhibitors of HCV NS3 protease, inhibitors of HCV polymerase, inhibitors of another target in the HCV life cycle, HIV inhibitors, HAV inhibitors and HBV inhibitors. Examples of such agents are provided in the Definitions section above.
Specific preferred examples of some of these agents are listed below:

- ■ antiviral agents: ribavirin and amantadine;
- ■ immunomodulatory agents: class I interferons, class II interferons and pegylated interferons;
- ■ inhibitor of another target in the HCV life cycle that inhibits a target selected from: NS3 helicase, HCV NS2/3 protease or internal ribosome entry site (IRES);
- ■ HIV inhibitors: nucleosidic inhibitors, non-nucleosidic inhibitors, protease inhibitors, fusion inhibitors and integrase inhibitors; or
- ■ HBV inhibitors: agents that inhibit HBV viral DNA polymerase or is an HBV vaccine.

As discussed above, combination therapy is contemplated wherein a compound of formula (1), or a pharmaceutically acceptable salt thereof, is co-administered with at least one additional agent selected from: an antiviral agent, an immunomodulatory agent, another inhibitor of HCV NS3 protease, an inhibitor of HCV polymerase, an inhibitor of another target in the HCV life cycle, an HIV inhibitor, an HAV inhibitor and an HBV inhibitor. Examples of such agents are provided in the Definitions section above. These additional agents may be combined with the compounds of this invention to create a single pharmaceutical dosage form. Alternatively these additional agents may be separately administered to the patient as part of a multiple dosage form, for example, using a kit. Such additional agents may be administered to the patient prior to, concurrently with, or following the administration of wherein a compound of formula (1), or a pharmaceutically acceptable salt thereof.
As used herein, the term "treatment" means the administration of a compound or composition according to the present invention to alleviate or eliminate symptoms of the hepatitis C disease and/or to reduce viral load in a patient.
As used herein, the term "prevention" means the administration of a compound or composition according to the present invention post-exposure of the individual to the virus but before the appearance of symptoms of the disease, and/or prior to the detection of the virus in the blood.

## Preferred embodiments

Preferably, a compound of formula I is as defined above wherein $\mathbf{R^1}$ is hydroxy or $NHSO_2\mathbf{R^{1A}}$ wherein $\mathbf{R^{1A}}$ is $(C_{1-6})$alkyl, $(C_{3-7})$cycloalkyl, or $\{(C_{1-6})$alkyl-$(C_{3-7})$cycloalkyl$\}$ which are all optionally substituted 1 -3 times with halo, nitro

or O-$(C_{1-6})$alkyl, or phenyl which is optionally substituted from 1 to 3 times with halo, nitro, $(C_{1-6})$alkyl or O-$(C_{1-6})$alkyl.

More preferably, a compound of formula I is as defined above wherein $\mathbf{R^1}$ is hydroxy or $NHSO_2\mathbf{R^{1A}}$ wherein $\mathbf{R^{1A}}$ is methyl, ethyl, cyclopropyl, cyclobutyl, cyclopentyl, cyclopropylmethyl, cyclohexylethyl, $CCl_3$, $CF_3$, phenyl, 2-fluorophenyl, or 4-methylphenyl.

Most preferably, a compound of formula I is as defined above wherein $\mathbf{R^1}$ is hydroxy or $NHSO_2\mathbf{R^{1A}}$ wherein $\mathbf{R^{1A}}$ is methyl, cyclopropyl, $CF_3$ or phenyl. Again most preferably, $\mathbf{R^{1A}}$ is cyclopropyl.

Most preferably, $\mathbf{R^1}$ is hydroxy.

Most preferably, $\mathbf{R^2}$ is cyclopentyl.

Included within the preferred embodiments of this invention are all compounds of formula I as presented in Table 1.

According to an alternate embodiment, the pharmaceutical composition of this invention may additionally comprise another anti-HCV agent. Examples of anti-HCV agents include, α- (alpha), β- (beta), δ- (delta), γ- (gamma) or ω- (omega) interferon, ribavirin and amantadine.

According to another alternate embodiment, the pharmaceutical composition of this invention may additionally comprise another inhibitor of HCV NS3 protease.

According to another alternate embodiment, the pharmaceutical composition of this invention may additionally comprise an inhibitor of HCV polymerase.

According to yet another alternate embodiment, the pharmaceutical composition of this invention may additionally comprise an inhibitor of other targets in the HCV life cycle, including but not limited to, helicase, NS2/3 protease or internal ribosome entry site (IRES).

The pharmaceutical composition of this invention may be administered orally, parenterally or via an implanted reservoir. Oral administration or administration by injection is preferred. The pharmaceutical composition of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intra-articular, intrasynovial, intrasternal, intrathecal, and intralesional injection or infusion techniques.

The pharmaceutical composition may be in the form of a sterile injectable preparation, for example, as a sterile injectable aqueous or oleaginous suspension. This suspension may be formulated according to techniques known in the art using suitable dispersing or wetting agents (such as, for example Tween 80) and suspending agents.

The pharmaceutical composition of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added.

Other suitable vehicles or carriers for the above noted formulations and compositions can be found in standard pharmaceutical texts, e.g. in "Remington's Pharmaceutical Sciences", The Science and Practice of Pharmacy, 19th Ed. Mack Publishing Company, Easton, Penn., (1995).

Dosage levels of between about 0.01 and about 100mg/kg body weight per day, preferably between about 0.1 and about 50mg/kg body weight per day of the protease inhibitor compound described herein are useful in a monotherapy for the prevention and treatment of HCV mediated disease. Typically, the pharmaceutical composition of this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (*w*/*w*). Preferably, such preparations contain from about 20% to about 80% active compound.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician. Generally, treatment is initiated with small dosages substantially less than the optimum dose of the peptide. Thereafter, the dosage is increased by small increments until the optimum effect under the circumstances is reached. In general, the compound is most desirably administered at a concentration level that will generally afford antivirally effective results without causing any harmful or deleterious side effects.

When the composition of this invention comprise a combination of a compound of formula I and one or more additional therapeutic or prophylactic agent, both the compound and the additional agent should be present at dosage levels of between about 10 to 100%, and more preferably between about 10 and 80% of the dosage normally administered

in a monotherapy regimen.

When these compounds or their pharmaceutically acceptable salts are formulated together with a pharmaceutically acceptable carrier, the resulting composition may be administered *in vivo* to mammals, such as man, to inhibit HCV NS3 protease or to treat or prevent HCV virus infection. Such treatment may also be achieved using a compound of this invention in combination with agents which include, but are not limited to: α-, β-, δ-, ω-, tau- or γ-interferon, ribavirin, amantadine; other inhibitors of HCV NS3 protease; inhibitors of HCV polymerase; inhibitors of other targets in the HCV life cycle, which include but not limited to, helicase, NS2/3 protease, or internal ribosome entry site (IRES); or combinations thereof. The additional agents may be combined with compounds of this invention to create a single dosage form. Alternatively these additional agents may be separately administered to a mammal as part of a multiple dosage form.

If the pharmaceutical composition comprises only a compound of this invention as the active component, such method may additionally comprise the step of administering to said mammal an agent selected from an immunomodulatory agent, an antiviral agent, a HCV NS3 protease inhibitor, an inhibitor of HCV polymerase or an inhibitor of other targets in the HCV life cycle such as helicase, NS2/3 protease or IRES. Such additional agent may be administered to the mammal prior to, concurrently with, or following the administration of the composition of this invention.

A compound of formula I set forth herein may also be used as a laboratory reagent. A compound of this invention may also be used to treat or prevent viral contamination of materials and therefore reduce the risk of viral infection of laboratory or medical personnel or patients who come in contact with such materials (e.g. blood, tissue, surgical instruments and garments, laboratory instruments and garments, and blood collection apparatuses and materials).

A compound of formula I set forth herein may also be used as a research reagent. A compound of formula I may also be used as positive control to validate surrogate cell-based assays or *in vitro* or *in vivo* viral replication assays.

Further details of the invention are illustrated in the following examples which are understood to be non-limiting with respect to the appended claims.

**Methodology**

In general, the compound of formula I and intermediates therefore are prepared by known methods using reaction conditions which are known to be suitable for the reactants. Several such methods are disclosed in WO 00/09543 and WO 00/09558.

The following scheme illustrates a convenient process using known methods for preparing a key intermediate of formula **6a** from acyclic intermediates:

## SCHEME 1

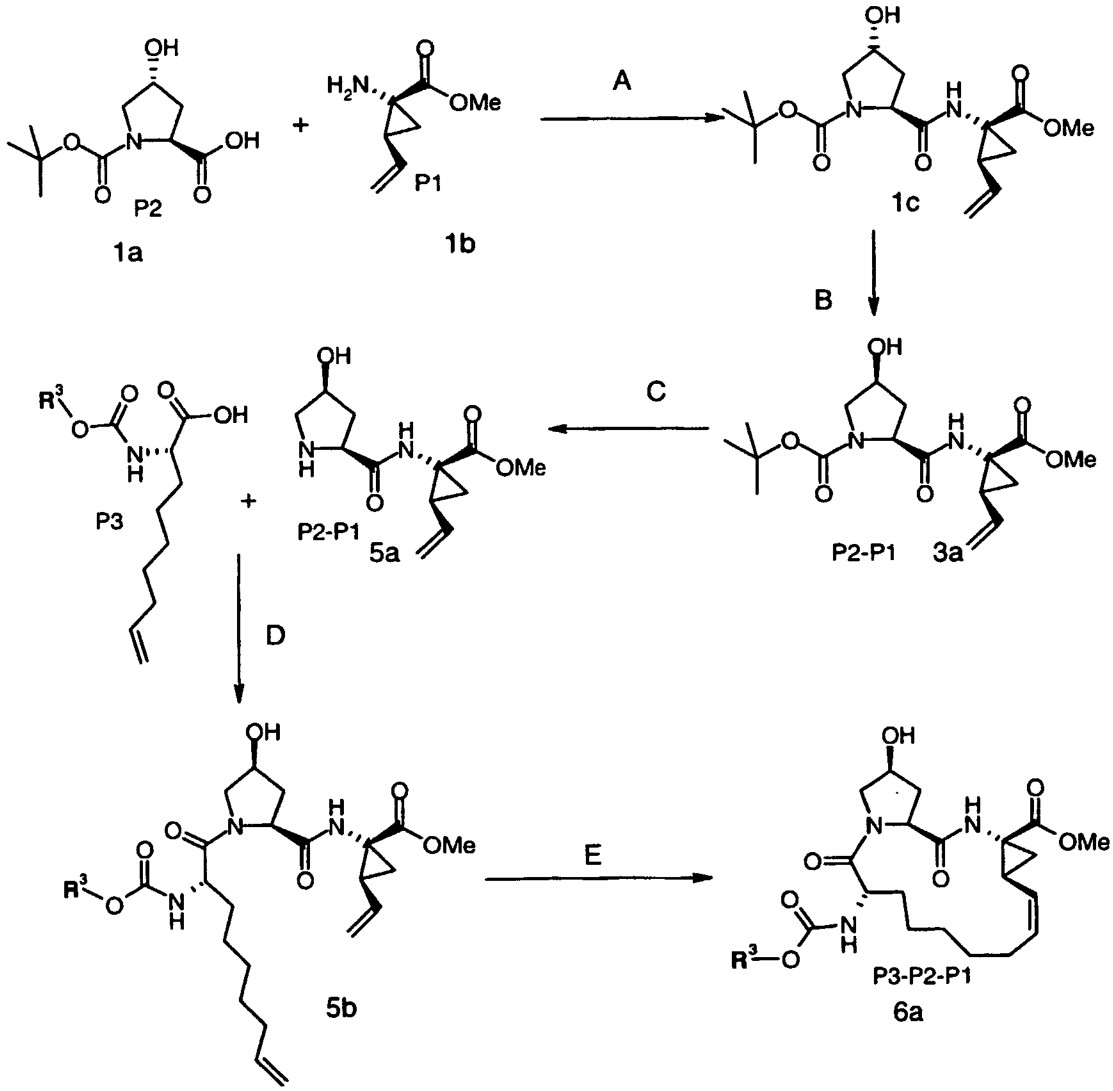


Scheme I:

**Steps A, C, D:** Briefly, the P1, P2, and P3 moieties can be linked by well known peptide coupling techniques generally disclosed in WO 00/09543 & WO 00/09558.

**Step B:** This step involves the inversion of configuration of the 4-hydroxy substituent. There are several ways in which this can be accomplished as will be recognized by persons skilled in the art. One example of a convenient method is the well known Mitsunobu reaction (Mitsunobu Synthesis 1981, January, 1-28; Rano et al. Tet. Lett. 1994, 36, 3779-3792; Krchnak et al. Tet. Lett. 1995, 36, 6193-6196). **Step E:** The formation of the macrocycle can be carried out via an olefin metathesis using a Ru-based catalyst such as the one reported by Miller, S.J.; Blackwell, H.E.; Grubbs, R.H. J. Am. Chem. Soc. 1996, 118, 9606-9614 **(a);** Kingsbury, J.S.; Harrity, J.P.A.; Bonitatebus, P.J.; Hoveyda, A.H. J. Am. Chem. Soc. 1999, 121, 791-799 **(b)** and Huang, J.; Stevens, E.D.; Nolan, S.P.; Petersen, J.L.; J. Am. Chem. Soc. 1999, 121, 2674-2678 (c) or as described in WO 00/59929. It will also be recognized that catalysts containing other transition metals such as Mo can be used for this reaction.

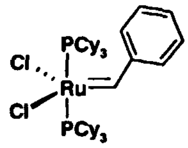

(a)

Grubbs' catalyst

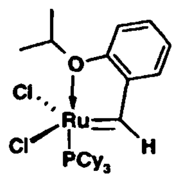

(b)

Horeyda's catalyst

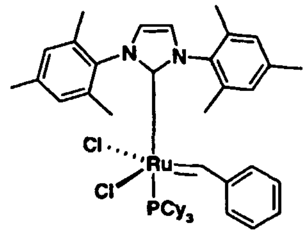

(c)

Nolan's catalyst

Subsequent conversion of the key intermediate of formula **6a** to the compounds of formula I of this invention is disclosed in detail in the examples hereinafter.

Compounds of formula I wherein $\mathbf{R^1}$ is $NHSO_2\mathbf{R^{1A}}$ as defined herein are prepared by coupling the corresponding acid of formula I (i.e. $\mathbf{R^1}$ is hydroxy) with an appropriate sulfonamide of formula $\mathbf{R^{1A}}\ SO_2NH_2$ in the presence of a coupling agent under standard conditions. Although several commonly used coupling agents can be employed, TBTU and HATU have been found to be practical. The sulfonamides are available commercially or can be prepared by known methods.

## EXAMPLES

The present invention is illustrated in further detail by the following examples. Other specific ways of synthesis or resolution can be found in WO 00/09543; WO 00/09558 & WO 00/59929.

Temperatures are given in degrees Celsius. Solution percentages express a weight to volume relationship, and solution ratios express a volume to volume relationship, unless stated otherwise. Nuclear magnetic resonance (NMR) spectra were recorded on a Bruker 400 MHz spectrometer; the chemical shifts (δ) are reported in parts per million and are referenced to the internal deuterated solvent unless otherwise indicated. The NMR spectra of all final compounds (inhibitors) was recorded in DMSO-$d_6$. Flash column chromatography was carried out on silica gel ($SiO_2$) according to Still's flash chromatography technique (W.C. Still et al., J. Org. Chem., 1978, 43, 2923).

Abbreviations used in the examples include Boc: *tert*-butyloxycarbonyl [$Me_3COC(O)$]; BSA: bovine serum albumin; CHAPS: 3-[(3-cholamidopropyl)-dimethylammonio]-1-propanesulfonate; $CH_2Cl_2$= DCM: methylene chloride; DEAD: diethylazodicarboxylate; DIAD: diisopropylazodicarboxylate; DIPEA: diisopropylethylamine; DMAP: dimethylaminopyridine; DMF: *N,N*-imethylformamide; DMSO: dimethylsulfoxide; (*S,S*)- Et-DUPHOS Rh (COD)OTf: (+)-1,2-bis (2*S*,5*S*)-2,5-diethylphospholano) benzene (cyctooctadiene) rhodinium (1) trifluoromethanesulfonate; EtOH: ethanol; EtOAc: ethyl acetate; ESMS: electrospray mass spectrometry; HATU: O-(7-azabenzotriazol-1-yl)-1,1,3,3-tetramethyluronium hexafluorophosphate; HPLC: high performance liquid chromatography; MS: mass spectrometry; MALDI-TOF: Matrix Assisted Laser Disorption Ionization-Time of Flight, FAB: Fast Atom Bombardment; Me: methyl; MeOH: methanol; R.T.: room temperature (18° -22°); TBTU: 2-(1H-benzotriazole-1-yl)-1,1,3,3-tetramethyluronium tetrafluoroborate; TFA: trifluoroacetic acid; THF: tetrahydrofuran; TLC: thin layer chromatography; Tris/HCl: tris(hydroxymethyl)aminomethane hydrochloride.

## EXAMPLE 1

Synthesis of dipeptide **1c:**

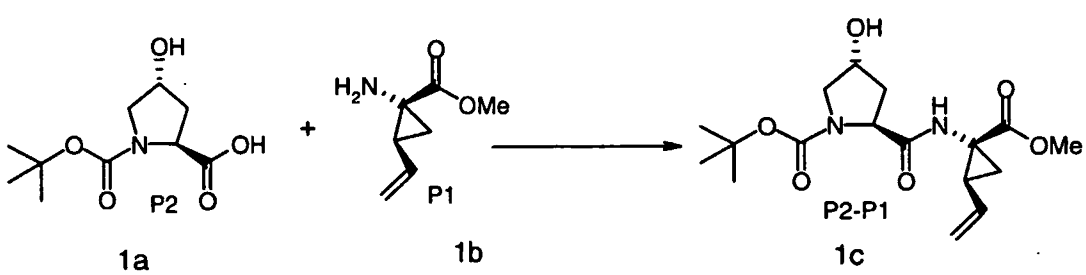


1a 1b 1c

A mixture of Boc-hydroxyproline **1a** (50.0g, 216mmol), (1*R*,2*S*)-vinyl-ACCA hydrochloride **1b** (42.25g, 238mmol), TBTU (76.36g, 238mmol) and DIPEA (113mL, 649mmol) in DMF (800mL) was stirred at R.T. under a nitrogen atmosphere. After 3.5h, the solvent was evaporated and the residue extracted with EtOAc and washed with hydrochloric acid (10%), saturated sodium bicarbonate and brine. The organic phase was then dried over magnesium sulfate, filtered and evaporated to afford an oil. Drying the oil overnight (18h) under high vacuum gave the dipeptide **1c** as a yellow foam (72.0 g, 94%, purity >95% by HPLC).

**EXAMPLE 2**

Synthesis of dipeptide **2a**

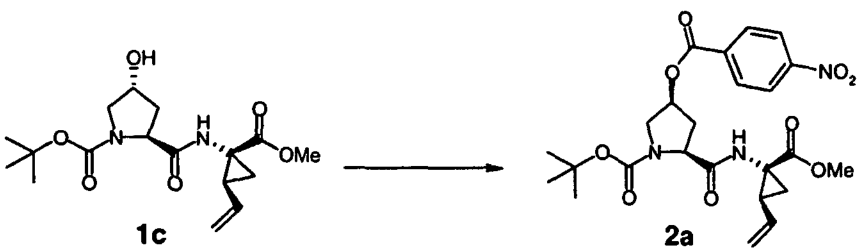

1c 2a

The dipeptide **1c** (72.0g, 203mmol), triphenylphosphine (63.94g, 243.8mmol, 1.2equiv.) and 4-nitrobenzoic acid (41.08g, 245.8mmol, 1.2equiv) were dissolved in dry THF (1.4L) and the stirred solution cooled to 0° under a nitrogen atmosphere. DEAD (38.4mL, 244mmol, 1.2equiv.) was then added dropwise over 45min and the reaction allowed to warm to R.T. After 4h, the solvent was evaporated and the residue divided into four portions. Each of these was chromatographed over fine silica gel (10-40 μm mesh, column diameter 12 cm, column length 16cm) using a gradient of 2 : 1 hexane/EtOAc to 1:1 hexane/EtOAc to pure EtOAc. Ester **2a** was obtained as an amorphous white solid after evaporation of the solvents and drying under high vacuum at 70° for 1 h (108.1 g, quantitative yield).

**EXAMPLE 3**

Synthesis of alcohol dipeptide **3a:**

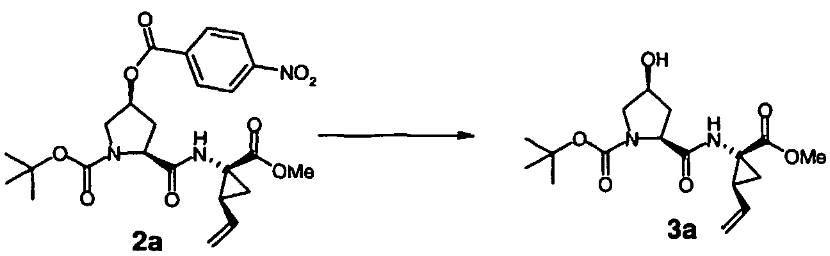

2a 3a

The nitrobenzoyl ester **2a** (108.1 g, 203.1 mmol) was dissolved in THF (1.0L) and the resulting solution cooled to 0°. A solution of lithium hydroxide monohydrate (10.66g, 253.9mmol) in water (225mL) was then added rapidly and the reaction stirred at 0° for 30min after which time the remaining base was neutralized with hydrochloric acid (1 N, 50.8mL). Additional acid was slowly added until the yellow color dissipated (7mL). The resulting mixture was then evaporated and the residue extracted with EtOAc (3 x 150mL). The extract was washed with saturated sodium bicarbonate (150mL) and brine (150mL). The organic phase was dried over magnesium sulfate-charcoal, filtered through diatomaceous earth and evaporated. Overnight drying of the residue under high vacuum yielded the alcohol **3a** as a colorless foam (70.1 g, 98%, purity >99% by HPLC).

**EXAMPLE 4**

Synthesis of (2*S*)-N-Boc-amino-non-8-enoic acid **(4g)**

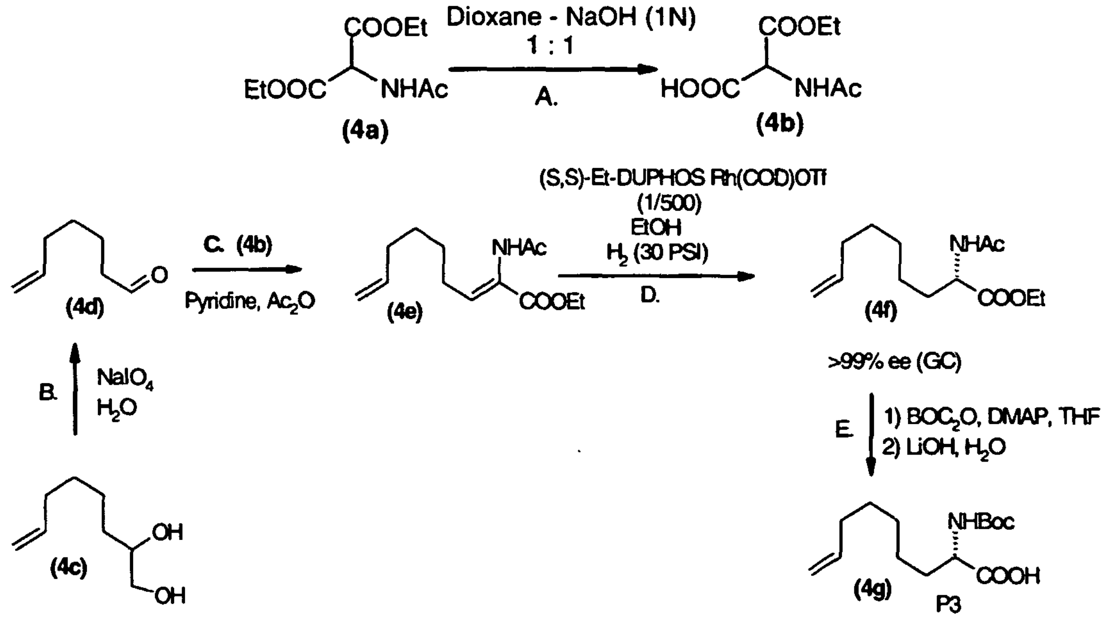


**Step A.** To a solution of commercially available diethyl 2-acetamidomalonate **4a** (100 g, 0.46mole) in dioxane (500mL) was added aqueous sodium hydroxide (1M, 1 eq., 460mL) dropwise over 30 to 45min The resulting mixture was left to stir for 16.5h, then dioxane was evaporated *in vacuo.* The resulting aqueous solution was extracted with three portions of 300mL of EtOAc and acidified to pH 1 with concentrated HCl. This solution was left to crystallize in an ice-water bath. After the appearance of a few crystals, the mixture was sonicated and an abundant precipitate appeared. Filtration and drying under vacuum afforded compound **4b,** (62.52g, 72% yield) as a white solid.

**Step B.** To a magnetically stirred emulsion of commercially available 7-octene-1,2-diol 4c (25g, 0.173mole) and $H_2O$ (100mL), in a 1 L round bottom flask, an aqueous solution of sodium periodate (40.7g, 0.190mole, 1.1eq., in 475mL $H_2O$) was added over a period of 20min (slightly exothermic). The resulting mixture was stirred at room temperature for an additional 1 h (completion of reaction confirmed by TLC). The mixture was then decanted in a separatory funnel and the aqueous layer was separated from the organic layer. The aqueous solution was saturated with NaCl, decanted and separated from the organic fraction once more. The two organic fractions were combined, dried with sodium sulfate and filtered over a cotton plug (in a Pasteur pipette) to give compound **4d** (15.135g, colorless oil, 78% yield). The aqueous solution was extracted with $CH_2Cl_2$, dried with anhydrous $MgSO_4$, and concentrated under vacuum (without heating, i.e. 6-heptanal b.p.153°C) to obtain an additional amount of compound **4d** (1.957g, colorless oil, 10% yield). Total yield 88%.

**Step C.** To solid ethyl 2-acetamidomalonate **4b** (7.57g, 40mmol) was added 6-heptenal **4d** (4.48g, 40mmol) in solution in pyridine (32mL, 10eq) over 1 min. The resulting solution was cooled in a 10° bath. Acetic anhydride (12mL, 3.2eq.) was added over 4min The resulting orange solution was stirred for 3h at R.T. and another portion of ethyl 2-acetamidomalonate **4b** (2.27g) was added. The resulting mixture was stirred at room temperature for an extra 11h. Ice (60mL) was then added and the solution was stirred for 1.5h, then the mixture was diluted with 250mL of water and extracted with two portions of diethyl ether. The etheral solution was washed with 1 N HCl, sat. $NaHCO_3$, dried $Na_2SO_4$, concentrated and purified by flash chromatography (EtOAc 40% / hexane) to give compound **4e** (4.8g, 50% yield) as a pale yellow oil.

**Step D.** To a degassed (argon bubbling for 30min) solution of Z-ethyl 2-acetamido-2,8-nonadienoate **4e** (8.38g, 35mmol) in dry ethanol (70mL) was added (*S,S*)-Et-DUPHOS Rh(COD)OTf (51 mg, (substrate/catalyst = 496). The mixture was put under 30 psi of hydrogen (after 4 vacuum-$H_2$ cycles) and stirred on a Parr shaker for 2h. The resulting mixture was evaporated to dryness to obtain the crude compound **4f,** which was used in the subsequent step without purification.

**Step E.** To a solution of crude (*S*)-ethyl 2-acetamido-8-nonenoate **4f** (7.3g, 30.3mmol) in THF (100mL), $Boc_2O$ (13.2g, 2eq.) and DMAP (740mg, 0.2eq) were added. The reaction mixture was heated at reflux for 2.5h. Subsequently, most of the THF solvent was evaporated, the crude mixture was diluted with $CH_2Cl_2$ and washed with 1 N HCl in order to

remove the DMAP. The organic layer was further extracted with saturated aqueous $NaHCO_3$, dried with anhydrous $Na_2SO_4$ and concentrated under vacuum. The crude product was then diluted with THF (50mL) and water (30mL), $LiOH.H_2O$ (2.54g, 2eq.) was added and the resulting mixture was stirred at R.T. for 25h (completion of the hydrolysis was confirmed by TLC). The reaction mixture was concentrated under vacuum to remove most of the THF solvent, and diluted with $CH_2Cl_2$. The resulting solution was washed with 1 N HCl, dried with anhydrous $Na_2SO_4$ and concentrated under vacuum. In order to remove minor impurities and excess $Boc_2O$, the crude product was purified by flash chromatography (using a solvent gradient from 100% hexane - 100% EtOAc as the eluent). The titled compound **4g** was obtained in high purity as a pale yellow oil (5.82g, 71% yield). $^1H$ NMR (DMSO, 400 MHz): δ 7.01 (d, J = 8 Hz, 1H), 5.79 (tdd, Jt = 6.7 Hz, Jd = 17.0, 10.2 Hz, 1 H), 5.00 (md, Jd = 17.0 Hz, 1H), 4.93 (md, Jd = 10.2 Hz, 1H), 3.83 (m, 1 H), 2.00 (q, J = 6.9 Hz, 2H), 1.65-1.5 (m, 2H), 1.38 (s, 9H), 1.35-1.21 (m, 6H).

**EXAMPLE 5**

Synthesis of tripeptide **5b**

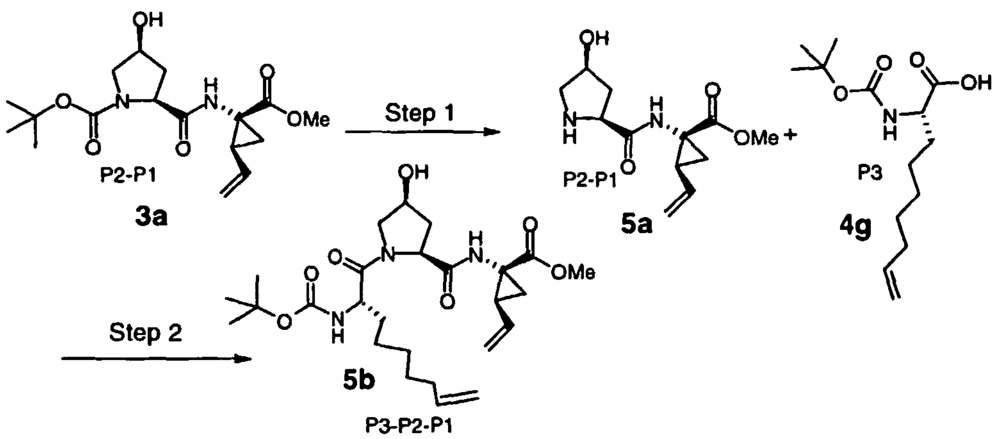


**Step 1 :** A solution of hydrogen chloride in dioxane (4N) was added to the Boc P2-P1 fragment **3a** (5.32g, 15.0mmol) resulting in a colorless solution. After 1 h of stirring at room temperature, the solvent was evaporated and the residue placed under high vacuum for 3h which afforded the hydrochloride salt of compound **5a** as an amorphous solid which was used as such.

**Step 2 :** DIPEA (2.6mL, 15mmol) was added to a mixture of the above prepared P1-P2 hydrochloride (15mmol) in dry DCM (100mL) resulting in a homogeneous solution. Separately, TBTU (5.30g, 16.5mmol, 1.1equiv.) was added to a stirred solution of C9-linker **4g** (4.07g, 15.0mmol) in dry DCM (130mL) resulting in partial dissolution of the reagent. DIPEA (2.6mL, 15mmol) was added resulting in an essentially homogeneous solution after 10min To this was then added the P1-P2 solution and DIPEA added until the reaction was basic (pH > 8 on wet litmus). After stirring under a nitrogen atmosphere for 5h, the solvent was evaporated and the residue extracted with EtOAc (2 x 250mL) and washed with dilute hydrochloric acid (0.05N, 400mL), water (400mL), and saturated sodium bicarbonate (400mL). The combined organic phases were then dried over magnesium sulfate, filtered and evaporated to a yellow syrup. The crude product was chromatographed over silica gel using 6:1 EtOAc / hexane to pure EtOAc as eluent, which afforded the desired tripeptide, diene **5b,** as a white foam (5.88g, 82%, purity >95% by HPLC).

**EXAMPLE 6**

synthesis of macrocyclic intermediate **6a:**

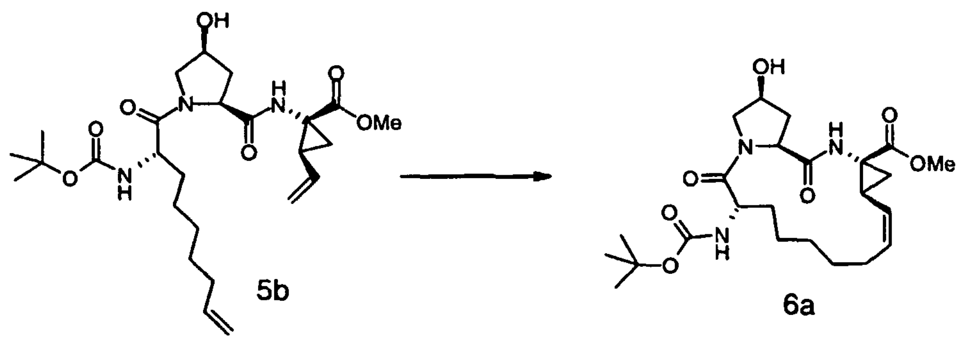

A solution of diene **5b** (4.0g, 7.88mmol) in dry DCM (800mL) was deoxygenated by bubbling Ar for 2h. Hoveyda's catalyst (262mg, 0.434mmol, 5.5mol %) was then added as a solid and the reaction was refluxed under an Ar balloon. After 28h, the red-orange solution was evaporated to an amorphous solid and then purified by flash column chromatography over silica gel. The initial solvent system was 10% EtOAc in $CH_2Cl_2$. Once the catalyst was eluted from the column, the solvent was changed to pure EtOAc. Elution of the catalyst from the column was evident from its color. The macrocyclic product **6a** was isolated as a colorless foam which was re-dissolved in $CH_2Cl_2$/hexane (-1:2). Evaporation of the solvent afforded a white powder (3.362g, 89% yield).
$^1$H NMR ($CDCl_3$, 400 MHz): δ 1.20-1.50 (m, 6H), 1.43 (s, 9H), 1.53 (dd, J = 9.5 & 5.4, 1 H), 1.61-1.70 (m, 1 H), 1.76-1.90 (m, 2H), 2.05-2.26 (m, 4H), 2.45 (d, J = 14.3, 1H), 3.67 (s, 3H), 3.71 (d, J = 11.1, 1H), 3.90 (dd, J = 11.1 & 4.3, 1H), 4.43-4.53 (m, 2H), 4.76 (d, J = 8.6, 1 H), 4.86 (bd, J = 9.8, 1 H), 5.20-5.23 (m, 2H), 5.57 (dt, J = 7.0 & 9.8, 1 H), 7.32 (bs, 1 H).

## EXAMPLE 7

Preparation of Thioureas **7**

Synthesis of thiourea **7a:**

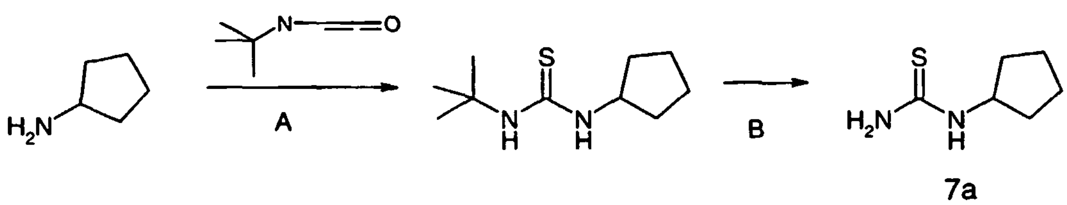


To a solution of *tert*-butyl isothiocyanate (5.0mL; 39.4mmol) in DCM (200mL) was added cyclopentylamine (4.67mL; 47.3mmol) followed by DIEA and the reaction mixture was stirred at R.T. for 2h. The mixture was diluted with EtOAc, washed with a 10% aqueous solution of citric acid (2x), saturated $NaHCO_3$ (2x), $H_2O$ (2x) and brine (1 x). The organic layer was dried over anhydrous $MgSO_4$, filtered and evaporated to yield N-*tert*-butyl - N'-cyclopentyl thiourea as a white solid (3.70g; 47% yield). The N-*tert*-butyl-N'-cyclopentyl thiourea (3.70g) was dissolved in concentrated HCl (46mL). The dark yellow solution was set to a gentle reflux. After 40min the reaction mixture was allowed to cool to R.T. and then cooled in ice and basified to pH 9.5 with solid and a saturated aqueous solution of $NaHCO_3$. The product was extracted into EtOAc (3x), the combined EtOAc extracts were washed with $H_2O$ (2x) and brine (1x). The organic layer was dried ($MgSO_4$), filtered and concentrated to obtain a beige solid (2.46g crude). Trituration of the crude material in hexane/ EtOAc 95 / 5 provided, after filtration, the N-cyclopentythiourea **7a** as a white solid (2.38; 90 % yield).
$^1$H NMR (400 MHz,DMSO-$d_6$): δ7.58 (bs, 1 H), 7.19 (bs, 1 H), 6.76 (bs, 1 H), 4.34 (bs, 1H), 1.92-1.79 (m,2H), 1.66-1.55 (m, 2H), 1.55-1.30 (m,4H). MS; es$^+$ 144.9(M + H)$^+$, es$^-$: 142.8 (M - H)$^-$.

Preparation of thiourea **7b**

Using the procedure described above and using commercially available cyclohexylamine (instead of cyclopentylamine) yielded thiourea **7b**

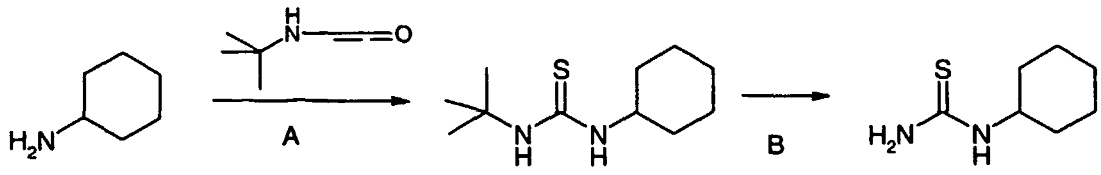


7b

## EXAMPLE 8

Synthesis Compound 101:

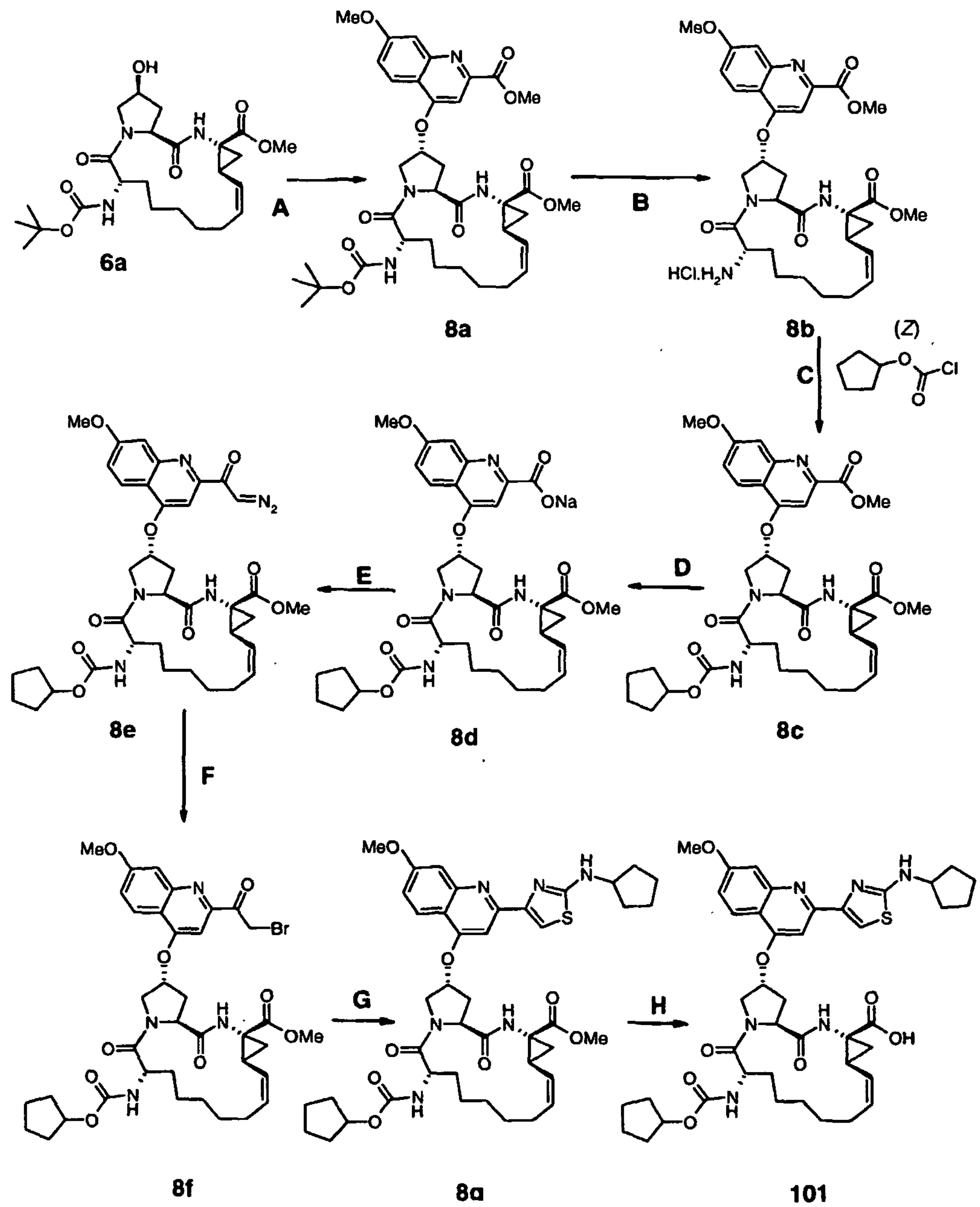


6a 8a 8b 8c 8d 8e 8f 8a 101

**Step A.** To a solution of the macrocyclic intermediate **6a** (13.05g, 27.2mmol, 1.0eq.), $Ph_3P$ (14.28g, 54.4mmol, 2.0eq) and 2-carboxymethoxy-4-hydroxy-7-methoxyquinoline (WO 00/09543; WO 00/09558 & WO 00/59929) (6.67g, 28.6mmol, 1.05eq) in THF (450mL) at 0°, DIAD (10.75mL, 54.6mmol, 2.0eq) was added dropwise over a period of 15min The ice bath was then removed and the reaction mixture was stirred at R.T. for 3h. After the complete conversion of starting material to products, the solvent was evaporated under vacuum, the remaining mixture diluted with EtOAc, washed with saturated $NaHCO_3$ (2x) and brine (1x), the organic layer was dried over anhydrous MgSO4, filtered and evaporated to dryness. Pure compound **7a** was obtained after flash column chromatography; the column was eluted first with hexane/EtOAc (50:50), followed by $CHCl_3$/EtOAc (95:5) to remove $Ph_3PO$ and DIAD byproducts and elution of the impurities was monitored by TLC. Finally, the desired product **8a** was eluted from the column with $CHCl_3$/EtOAc (70:30). Usually, the chromatography step had to be repeated 2-3 times before compound **8a** could be isolated in high purity as a white solid with an overall yield of 68% (12.8g, 99.5% pure by HPLC).

**Step B.** To a solution of the Boc-protected intermediate **8a** (1.567g) in $CH_2Cl_2$ (15mL), 4N HCl in dioxane (12mL) was added. The reaction mixture was stirred at R.T. for 1 h. [In the event that a thick gel would form half way through the reaction period, an additional 10mL $CH_2Cl_2$ was added.] Upon completion of the deprotection the solvents were evaporated to dryness to obtain a yellow solid and a paste like material. The mixture was redissolved in approximately 5% MeOH in $CH_2Cl_2$ and reevaporated to dryness under vacuum to obtain compound **8b** as a yellow solid, which was used in the next step without any purification.

**Step C.** To a solution of cyclopentanol (614 μL, 6.76mmol) in THF (15mL), a solution of phosgene in toluene (1.93M, 5.96mL, 11.502mmol) was added dropwise and the mixture was stirred at R.T. for 2h to form the cyclopentyl chloroformate reagent (z). After that period, approximately half of the solvent was removed by evaporation under vacuum. The remaining light yellow solution was diluted by the addition of $CH_2Cl_2$ (5mL) and concentrated to half of its original volume, in order to assure the removal of all excess phosgene. The above solution of the cyclopentyl chloroformate reagent was further diluted with THF (15mL) and added to the amine-2HCl salt **8b.** The mixture was cooled to 0° in an ice bath, the pH was adjusted to ~8.5-9 with the addition of $Et_3N$ (added dropwise) and the reaction mixture was stirred at 0° for 1 h. After that period, the mixture was diluted with EtOAc, washed with water (1x), saturated $NaHCO_3$ (2x), $H_2O$ (2x) and brine (1x). The organic layer was dried over anhydrous $MgSO_4$, filtered and evaporated under vacuum to obtain a yellow-amber foam. Dimethyl ester 8c was obtained as a white foam after purification by flash column chromatography (using a solvent gradient from 30% hexane to 20% hexane in EtOAc as the eluent) in 80% yield (1.27g) and >93% purity.

**Step D.** The dimethyl ester **8c** (1.17g) was dissolved in a mixture of THF/MeOH/$H_2O$ (20mL, 2:1:1 ratio), and an aqueous solution of NaOH (1.8mL, 1 N, 1 eq.) was added. The reaction mixture was stirred at R.T. for 1 h before it was evaporated to dryness to obtain the sodium salt **8d** as a white solid (~1.66mmol). Compound **8d** was used in the next step without purification.

**Step E.** The crude sodium salt **8d** (1.66mmol) was dissolved in THF (17mL), $Et_3N$ was added and the mixture was cooled to 0° in an ice bath. Isobutylchloroformate (322 μl, 2.5mmol) was added dropwise and the mixture was stirred at 0° for 75min After that period, diazomethane (15mL) was added and stirring was continued at 0° for 30min and then at R.T. for an additional 1 h. Most of the solvent was evaporated to dryness under vacuum, the remaining mixture was diluted with EtOAc, washed with saturated $NaHCO_3$ (2x), $H_2O$ (2x) and brine (1x), dried over anhydrous $MgSO_4$, filtered and evaporated to dryness to obtain compound **8e** as a light yellow foam (1.2g, ~1.66mmol). The diazoketone intermediate **8e** was used in the next step without purification.

**Step F.** A solution of the diazoketone **8e** (1.2g, 1.66mmol) dissolved in THF (17mL) was cooled to 0° in an ice bath. A solution of aqueous HBr (48%, 1.24mL) was added dropwise and the reaction mixture was stirred at 0° for 1 h. The mixture was then diluted with EtOAc, washed with saturated $NaHCO_3$ (2x), $H_2O$ (2x) and brine (1 x). The organic layer was dried over anhydrous $MgSO_4$, filtered and evaporated to dryness to obtain the α-bromoketone intermediate **8f** as a light yellow foam (~1.657mmol).

**Step G.** To a solution of the bromoketone **8f** (2.51 g, 3.27 mmol) in isopropanol (105mL), cyclopentylthiourea **7a** (565mg, 3.92mmol) was added and the reaction mixture was placed in a preheated oil bath at 70° where it was stirred for 1.5h. The isopropanol was then removed under vacuum and the product dissolved in EtOAc. The solution was washed with saturated $NaHCO_3$, water and brine, the organic layer was dried over anhydrous $MgSO_4$, filtered and evaporated to afford the crude product **8g** (1.35g) as a light yellow solid. The crude product was purified by flash chromatography in silica gel (1:1hexane/EtOAc) to afford 2.12mg of an off-white solid (80% yield).

**Step H.** The methyl ester **8g** (1.82g, 2.2mmol) was dissolved in a solution of THF/MeOH/$H_2O$(38/20/18mL) and saponified with LiOH. $H_2O$(935mg, 22.3mmol). The hydrolysis reaction was carried out over 18h at R.T. Thereafter, the solution was evaporated to dryness to give an off-white paste. The paste was diluted with EtOAc and brine. The mixture was adjusted to pH 6 with 1 N HCl. The EtOAc layer was separated and the aqueous layer was extracted twice with EtOAc. The combined EtOAc extracts were washed with deionized water (2X) and brine (1X), dried (MgSO4), and evaporated to afford the cyclic tripeptide compound **101** as a yellow solid (1.76g; 99% yield).

**Conversion to Na Salt:**

Compound **8h** (106mg; 0.132mmol) was dissolved in MeOH (20mL) and a 0.01 N solution of NaOH (13.2mL) was added. The clear yellow solution was diluted with water, frozen and lyophilized to yield compound **8h** Na salt of as a yellow-white amorphous solid (106mg; 97 % yield).
M.S.(electrospray) : 799.3 (M-H)- 801.4 (M+H) Reverse Phase HPLC Homogeneity (0.06 % TFA ; $CH_3CN$ : $H_2O$) : 99 %. $^1H$ NMR (400 MHz,DMSO-$d_6$): δ 8.02 (d, J= 9.2 Hz, 1H), 7.90 (d, J = 6.4 Hz, 1H), 7.76 (s, 1 H), 7.44 (bs, 2H), 7.27 (d, J = 1.9 Hz, 1H), 7.11 (d, J = 7.0 Hz, 1H), 7.03 (d, J = 9.2 Hz, 1 H), 5.48 (dd, J = 18.4, 9.9 Hz, 1H), 5.43 (bs, 1 H), 5.15 (dt, J = 17.8, 7.63 Hz, 1 H), 4.70 (bs, 1 H), 4.49-4.34 (m, 2H), 4.34-4.25 (m, 1 H), 4.13-4.03 (m, 1 H), 3.99-3.86 (m, 1H), 3.90 (s, 3H), 2.58-2.44 (m, 1 H), 2.42-2.32 (m, 1 H), 2.15-1.93 (m, 4H), 1.83-1.14 (m, 24H), 1.14-1.12 (m, 1H).

**EXAMPLE 9**

Using the same procedure as described in Example 8 but, in step G, using N-cyclohexylthiourea **7b,** gave the sodium salt of compound **102.**

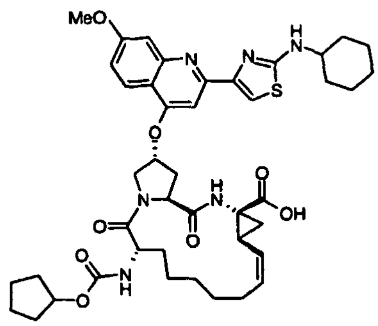

**Compound 102**

$^1H$ NMR (400 MHz,DMSO-$d_6$): δ8.02 (d, J = 9.2 Hz, 1 H), 7.86 (bs, 1H), 7.78 (d, J = 7.3 Hz, 1H), 7.43 (s, 2H), 7.27 (d, J = 2.2 Hz, 1 H), 7.12 ( d, J = 6.9 Hz, 1H), 7.03 ( dd, J = 9.2, 1.9 Hz, 1 H), 5.57-5.40 (m, 1H), 5.40 (s, 1H), 5.26-5.17 (m, 1 H), 4.70 (bs, 1H), 4.52-4.35 (m, 2H), 4.29-4.23 (m, 1 H), 4.18-4.00 (m, 1 H), 3.90 (s, 3H), 3.87-3.65 (m, 1H), 2.42-2.32 (m, 1H), 2.19-2.10 (m, 1 H), 2.07-1.96 (m, 3H), 1.82-0.95 (m, 28H). MS; $es^{+}$: 815.4$(M + H)^+$, $es^-$: 813.4 $(M - H)^-$.

**EXAMPLE 10**

NS3-NS4A protease assay

The enzymatic assay used to evaluate the present compound is described in WO 00/09543 and WO 00/59929.

**EXAMPLE 11**

Cell Based HCV RNA Replication Assay

Cell Culture:

Huh7 cells that stably maintain a subgenomic HCV replicon were established as previously described (Lohman et al., 1999. Science 285: 110-113) and designated as the S22.3 cell-line (WO 02/052015). S22.3 cells are maintained in Dulbecco's Modified Earle Medium (DMEM) supplemented with 10% FBS and 1 mg/mL neomycin (Standard Medium). During the assay, DMEM medium supplemented with 10% FBS, containing 0.5% DMSO and lacking neomycin was used (Assay Medium). 16 hours prior to compound addition, S22.3 cells are trypsinized and diluted to 50 000 cells/ml in Standard Medium. 200μL (10 000 cells) are distributed into each well of a 96-well plate. The plate was then incubated at 37° with 5% $CO_2$ until the next day.

Reagents and Materials:

| Product | Company | Catalog # | Storage |
|---|---|---|---|
| DMEM | Wisent Inc. | 10013CV | 4°C |
| DMSO | Sigma | D-2650 | R.T. |
| Dulbecco's PBS | Gibco-BRL | 14190-136 | R.T. |
| Fetal Bovine Serum | Bio-Whittaker | 14-901 F | -20°C/4°C |
| Neomycin (G418) | Gibco-BRL | 10131-027 | -20°C/4°C |
| Trypsin-EDTA | Gibco-BRL | 25300-054 | -20°C/4°C |
| 96-well plates | Costar | 3997 | R.T. |
| PVDF 0.22μm Filter Unit | Millipore | SLGV025LS | R.T. |
| Deep-Well Titer Plate Polypropylene | Beckman | 267007 | R.T. |

Preparation of Test Compound

10μL of test compound (in 100% DMSO) was added to 2 ml of Assay Medium for a final DMSO concentration of 0.5% and the solution was sonicated for 15min and filtered through a 0.22μM Millipore Filter Unit. 900μl was transferred into row A of a Polypropylene Deep-Well Titer Plate. Rows B to H, contain 400μL aliquots of Assay Medium (containing 0.5% DMSO), and are used to prepare serial dilutions (1/2) by transferring 400μl from row to row (no compound was included in row H).

Application of test compound to cells

Cell culture medium was aspirated from the 96-well plate containing the S22.3 cells. 175μL of assay medium with the appropriate dilution of test compound was transferred from each well of the compound plate to the corresponding well of the cell culture plate (row H was used as the "No inhibition control"). The cell culture plate was incubated at 37° with 5% $CO_2$ for 72h.

Extraction of Total Cellular RNA

Following the 72h incubation period, the total cellular RNA was extracted from the S22.3 cells of the 96-well plate using the RNeasy 96 kit (Qiagen®, RNeasy Handbook. 1999.). Briefly, assay medium was completely removed from cells and 100 μL of RLT buffer (Qiagen®) containing 143 mM β-mercaptoethanol was added to each well of the 96-well cell-culture plate. The microplate was gently shaken for 20 sec. 100 μL of 70% ethanol was then added to each microplate well, and mixed by pipetting. The lysate was removed and applied to the wells of a RNeasy 96 (Qiagen®) plate that was placed on top of a Qiagen® Square-Well Block. The RNeasy 96 plate was sealed with tape and the Square-Well Block with the RNeasy 96 plate was loaded into the holder and placed in a rotor bucket of a 4K15C centrifuge. The sample was centrifuged at 6000 rpm (-5600 x g) for 4min at room temperature. The tape was removed from the plate and 0.8 ml of Buffer RW1 (Qiagen® RNeasy 96 kit) was added to each well of the RNeasy 96 plate. The RNeasy 96 plate was sealed with a new piece of tape and centrifuged at 6000 rpm for 4min at room temperature. The RNeasy 96 plate was placed on top of another clean Square-Well Block, the tape removed and 0.8 ml of Buffer RPE (Qiagen® RNeasy 96 kit) was added to each well of the RNeasy 96 plate. The RNeasy 96 plate was sealed with a new piece of tape and centrifuged at 6000 rpm for 4min at room temperature. The tape was removed and another 0.8 ml of Buffer RPE (Qiagen® RNeasy 96 kit) was added to each well of the RNeasy 96 plate. The RNeasy 96 plate was sealed with a new piece of tape and centrifuged at 6000 rpm for 10min at room temperature. Tape was removed, the RNeasy 96 plate was placed on top of a rack containing 1.2-mL collection microtubes. The RNA was eluted by adding 50 μL of RNase-free water to each well, sealing plate with a new piece of tape and incubated for 1min at room temperature. The plate was then centrifuged at 6000 rpm for 4min at room temperature. The elution step was repeated with a second volume of 50 μl RNase-free water. The microtubes with total cellular RNA are stored at -70°.

Quantification of Total Cellular RNA

RNA was quantified on the STORM® system (Molecular Dynamics®) using the RiboGreen® RNA Quantification Kit (Molecular Probes®). Briefly, the RiboGreen reagent was diluted 200-fold in TE (10mM Tris-HCl pH =7.5, 1 mM

EDTA). Generally, 50μL of reagent was diluted in 10mL TE. A Standard Curve of ribosomal RNA was diluted in TE to 2μg/mL and pre-determined amounts (100, 50, 40, 20, 10, 5, 2 and 0μL) of the ribosomal RNA solution are then transferred in a new 96-well plate (COSTAR # 3997) and the volume was completed to 100μL with TE. Generally, column 1 of the 96-well plate was used for the standard curve and the other wells are used for the RNA samples to be quantified. 10μL of each RNA sample that was to be quantified, was transferred to the corresponding well of the 96-well plate and 90μL of TE was added. One volume (100μL) of diluted RiboGreen reagent was added to each well of the 96-well plate and incubated for 2 to 5 minutes at room temperature, protected from light (a 10 μL RNA sample in a 200 μL final volume generates a 20 X dilution). The fluorescence intensity of each well was measured on the STORM® system (Molecular Dynamic®). A standard curve was created on the basis of the known quantities of the ribosomal RNA and the resulting fluorescent intensities. The RNA concentration in the experimental samples was determined from the standard curve and corrected for the 20X dilution.

Reagents and Materials:

| Product | Company | Catalog # | Storage |
|---|---|---|---|
| DEPC | Sigma | D5758 | 4˚C |
| EDTA | Sigma | E5134 | R.T. |
| Trizma-Base | Sigma | T8524 | R.T. |
| Trizma-HCl | Sigma | T7149 | R.T. |
| Collection Tube Strips | Qiagen | 19562 | R.T. |
| Ribogreen RNA Quantitation Kit | Molecular Probe | R11490 | -20˚C |
| RNeasy 96 Kit | Qiagen | 74183 | R.T. |
| Square-Well Blocks | Qiagen | 19573 | R.T. |

Real-Time R.T.-PCR

The Real-Time R.T.-PCR was performed on the ABI Prism 7700 Sequence Detection System using the TaqMan EZ R.T.-PCR Kit from (Perkin-Elmer Applied Biosystems®). R.T.-PCR was optimized for the quantification of the 5'IRES of HCV RNA by using the Taqman technology (Roche Molecular Diagnostics Systems) similar to the technique previously described (Martell et al., 1999. J. Clin. Microbiol. 37: 327-332). The system exploits the 5'-3' nucleolytic activity of AmpliTaq DNA polymerase. Briefly, the method utilizes a dual-labeled fluorogenic hybridization probe (PUTR Probe) that specifically anneals to the template between the PCR primers (primers 8125 and 7028). The 5' end of the probe contains a fluorescent reporter (6-carboxyfluorescein [FAM]) and the 3' end contains a fluorescent quencher (6-carboxytetramethylrhodamine [TAMRA]). The FAM reporter's emission spectrum was suppressed by the quencher on the intact hybridization probe. Nuclease degradation of the hybridization probe releases the reporter, resulting in an increase in fluorescence emission. The ABI Prism 7700 sequence detector measures the increase in fluorescence emission continuously during the PCR amplification such that the amplified product was directly proportion to the signal. The amplification plot was analysed early in the reaction at a point thatrepresents the logarithmic phase of product accumulation. A point representing a defined detection threshold of the increase in the fluorescent signal associated with the exponential growth of the PCR product for the sequence detector was defined as the cycle threshold ($C_T$). $C_T$ values are inversely proportional to the quantity of input HCV RNA; such that under identical PCR conditions, the larger the starting concentration of HCV RNA, the lower the $C_T$. A standard curve was created automatically by the ABI Prism 7700 detection system by plotting the $C_T$ against each standard dilution of known HCV RNA concentration.

Reference samples for the standard curve are included on each R.T.-PCR plate. HCV Replicon RNA was synthesized (by T7 transcription) *in vitro,* purified and quantified by $OD_{260}$. Considering that 1μg of this RNA = 2.15 X $10^{11}$ RNA copies, dilutions were made in order to have $10^8$, $10^7$, $10^6$, $10^5$, $10^4$, $10^3$ or $10^2$ genomic RNA copies / 5μL. Total cellular Huh-7 RNA was also incorporated with each dilution (50ng / 5μL). 5μL of each reference standard (HCV Replicon + Huh-7 RNA) was combined with 45μL of Reagent Mix, and used in the Real-Time R.T.-PCR reaction.

The Real-Time R.T.-PCR reaction was set-up for the experimental samples that were purified on RNeasy 96 -well plates by combining 5μl of each total cellular RNA sample with 45μL of Reagent Mix.

Reagents and Materials:

| Product | COMPANY | Catalog # | Storage |
|---|---|---|---|
| TaqMan EZ R.T.-PCR Kit | PE Applied Biosystems | N808-0236 | -20˚C |
| MicroAmp Optical Caps | PE Applied Biosystems | N801-0935 | R.T. |
| MicroAmp Optical 96-Well Reaction Plate | PE Applied Biosystems | N801-0560 | R.T. |

Reagent Mix preparation:

| Component | Volume for one sample (μL) | Volume for One Plate (μL) (91 samples + Dead Volume) | Final conc. |
|---|---|---|---|
| Rnase-free water | 16.5 | 1617 | |
| 5X TaqMan EZ buffer | 10 | 980 | 1 X |
| $Mn(OAc)_2$ (25mM) | 6 | 588 | 3mM |
| dATP (10mM) | 1.5 | 147 | 300μM |
| dCTP (10mM) | 1.5 | 147 | 300μM |
| dGTP (10mM) | 1.5 | 147 | 300μM |
| dUTP (20mM) | 1.5 | 147 | 600μM |
| Forward Primer (10μM) | 1 | 98 | 200nM |
| Reverse Primer (10μM) | 1 | 98 | 200nM |
| PUTR probe (5μM) | 2 | 196 | 200nM |
| rTth DNA polymerase (2.5 U/μL) | 2 | 196 | 0.1U/μL |
| AmpErase UNG (1U/μL) | 0.5 | 49 | 0.01 U/μL |
| Total Volume | 45 | 4410 | |
| **Forward Primer Sequence (SEQ ID. 1):** 5' - ACG CAG AAA GCG TCT AGC CAT GGC GTT AGT - 3'<br>**Reverse Primer Sequence (SEQ ID NO. 2):** 5' - TCC CGG GGC ACT CGC AAG CAC CCT ATC AGG - 3'<br>Note: Those primers amplify a region of 256-nt present within the 5' untranslated region of HCV.<br>**PUTR Probe Sequence (SEQ ID NO. 3):** 6FAM - TGG TCT GCG GAA CCG GTG AGT ACA CC - AMRA | | | |

No Template Controls (NTC): On each plate, 4 wells are used as "NTC". For these controls, 5μl of water are added to the well in place of RNA.

Thermal Cycling Conditions:

| | | | |
|---|---|---|---|
| 50°C | 2min | | |
| 60°C | 30min | | |
| 95°C | 5min | | |
| | 95°C | 15 sec | for 2 cycles |
| 60°C | 1min | | |
| | 90°C | 15 sec | for 40 cycles |
| 60°C | 1min | | |

Following the termination of the R.T.-PCR reaction the data analysis requires setting of threshold fluorescence signal for the PCR plate and a standard curve was constructed by plotting the Ct value versus RNA copy number used in each reference reaction. The Ct values obtained for the assay samples are used to interpolate an RNA copy number based on the standard curve.

Finally, the RNA copy number was normalized (based on the RiboGreen RNA quantification of the total RNA extracted from the cell culture well) and expressed as genome equivalents / µg of total RNA [ge/µg].

The RNA copy number [g.e./µg] from each well of the cell culture plate was a measure of the amount of replicating HCV RNA in the presence of various concentrations of inhibitor. The % inhibition was calculated with the following equation:

$$100 - [(g.e./\mu g\ inh)/( g.e./\mu g\ ctl) x\ 100].$$

A non-linear curve fit with the Hill model was applied to the inhibition-concentration data, and the 50% effective concentration ($EC_{50}$) was calculated by the use of SAS software (Statistical Software System; SAS Institute, Inc. Cary, N.C.).

**EXAMPLE 12**

Specificity assays

The specificity assays used to evaluate the selectivity of this compound are described in WO 00/09543.

**EXAMPLE 13**

Pharmacokinetic properties

The present compounds also show good pharmacokinetic properties such as detectable plasma levels in the rat at 1 hour and 2h after an oral dose of 5mg/kg.

More explicitly, the following assay, an *in vivo* oral absorption screen, was used to determine plasma levels of test compounds in a rat after oral administration:

Materials and Methods:

**1. Method used to pool compounds ("cassette selection"):**

The selection of compounds to be pooled into a "cassette" was based on their structural similarity and physicochemical properties. A solid phase extraction method applicable to all the selected compounds was established. Based on the initial testing where each compound was spiked into rat plasma and run through HPLC or HPLC/MS at a concentration of 0.5 µM, the retention time, ionic mass, and the possible separation among compounds by HPLC and/or HPLC/MS were used as basis for pooling 3-4 compounds into one "cassette".

**2. Oral vehicle and compound preparation:**

Each "cassette" contains 3-4 compounds at 5 or 4mg/kg for each compound. The cassettes were prepared as an oral suspension in 0.5% aqueous methylcellulose and 0.3% of polyoxyethylene (20) sorbiton monooleate (Tween-80). The dosing volume was 10ml/kg via oral gavage.

**3. Dosing and plasma sampling:**

Male Sprague Dawley rats were fasted overnight in individual cages, with access to aqueous 10% dextrose. Two rats were dosed with each "cassette". Plasma samples (~1 ml) were collected at 1 and 2h post-dosing from the 2 rats and pooled for extraction and analysis.

**4. Compound extraction and analysis:**

From each cassette, plasma samples at 1 and 2h, blank plasma, blank plasma spiked with all the compounds at 0.5 μM of each, are extracted by the solid phase extraction method. Samples were analyzed by HPLC and HPLC/MS for comparison purpose. Plasma concentrations are estimated based on the single concentration of 0.5 μM standard.

**TABLE 1**

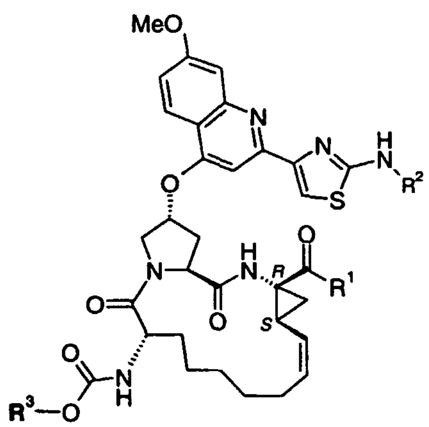

| **Cpd #** | $R^1$ | $R^2$ | $R^3$ | **MS** (M-H)- |
|---|---|---|---|---|
| **101** | OH | cyclopentyl | cyclopentyl | 799.3 |
| **102** | OH | cyclohexyl | cyclopentyl | 813.4 |
| **103** | NH-$SO_2$-cyclopropyl | cyclopentyl | cyclopentyl | |

When the compounds of this invention were evaluated the preceding enzymatic and cell based assays, the compounds were found to be highly active. More specifically, the compounds had $IC_{50}$'s below 0.01 μM in the NS3-NS4A protease assay, and $EC_{50}$'s below 0.01 μM in the cell based HCV RNA replication assay.

When the compounds were evaluated in the specificity assays, the compounds of formula I were found to be selective in that they do not show significant inhibition in the Human Leukocyte Elastase and Cathepsin B assays.

When the compounds were evaluated in pharmacokinetic assay in the rat, a dose of 5 mg/kg in Methocel: Tween20® (0.5%:0.3%) gave unexpectedly good plasma concentration. Compounds **101** and **102** gave area under the curve (AUC) of 16 and 18 μM-hr respectively, compared to their closest analogs which had AUC ranging from 0.8 to 4.5 μM-hr. This unexpected and significant increase in compound uptake in rat plasma makes these compounds particularly interesting as potential drugs that may be administered orally.

## Claims

**1.** A compound of formula I:

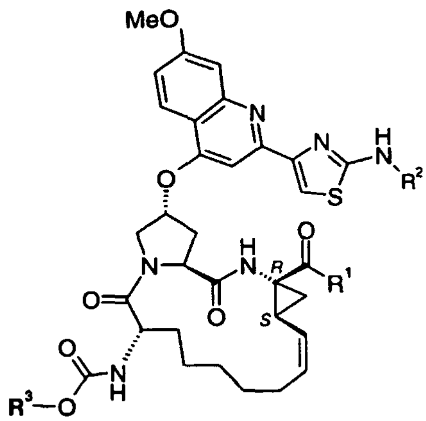

(I)

wherein $R^1$ is hydroxy or $NHSO_2R^{1A}$ wherein $R^{1A}$ is $(C_{1-8})$alkyl, $(C_{3-7})$cycloalkyl or {$(C_{1-6})$alkyl-$(C_{3-7})$cycloalkyl}, which are all optionally substituted from 1 to 3 times with halo, cyano, nitro, $O(C_{1-6})$alkyl, amido, amino or phenyl, or $R^{1A}$ is $C_6$ or $C_{10}$ aryl which is optionally substituted from 1 to 3 times with halo, cyano, nitro, $(C_{1-6})$alkyl, $O(C_{1-6})$ alkyl, amido, amino or phenyl; $R^2$ is $(C_{5-6})$ cycloalkyl and $R^3$ is cyclopentyl; or a pharmaceutically acceptable salt thereof.

2. The compound according to claim 1 wherein $R^1$ is hydroxy or $NHSO_2R^{1A}$ wherein $R^{1A}$ is $(C_{1-6})$alkyl, $(C_{3-7})$cycloalkyl or {$(C_{1-6})$alkyl-$(C_{3-7})$cycloalkyl}, which are all optionally substituted from 1 to 3 times with halo, nitro or $O(C_{1-6})$alkyl, or phenyl which is optionally substituted from 1 to 3 times with halo, nitro, $(C_{1-6})$alkyl or $O(C_{1-6})$alkyl.

3. The compound according to claim 2 wherein $R^1$ is $NHSO_2R^{1A}$ wherein $R^{1A}$ is methyl, ethyl, cyclopropyl, cyclobutyl, cyclopentyl, cyclopropylmethyl, cyclohexylethyl, $CCl_3$, $CF_3$, phenyl, 2-fluorophenyl, or 4-methylphenyl.

4. The compound according to claim 3 wherein $R^{1A}$ is cyclopropyl.

5. The compound according to claim 1 or 2, wherein $R^1$ is hydroxy.

6. The compound according to any one of claims 1 to 5 wherein $R^2$ is cyclopentyl.

7. The compound according to claim 1, having the following formula 101:

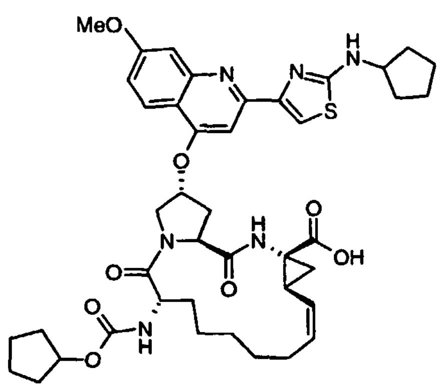

(101).

8. The compound according to claim 1, having the following of formula 102:

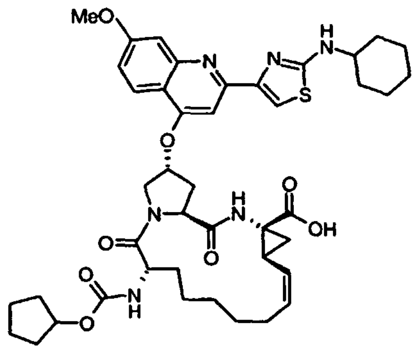

**(102).**

**9.** The compound according to claim 1, having the following formula 103:

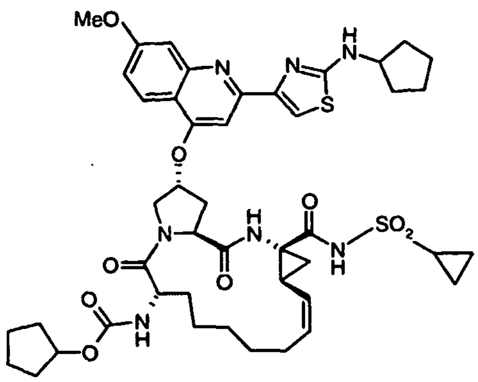

**(103).**

**10.** A pharmaceutical composition comprising an anti-hepatitis C virally effective amount of a compound of formula I according to any one of claims 1 to 9, or a pharmaceutically acceptable salt thereof, in admixture with a pharmaceutically acceptable carrier medium or auxiliary agent.

**11.** The pharmaceutical composition according to claim 10, further comprising a therapeutically effective amount of one or more other anti-HCV agent.

**12.** The pharmaceutical composition according to claim 11, wherein said other anti-HCV agent is selected from: α-interferon or pegylated α-interferon.

**13.** The pharmaceutical composition according to claim 11, wherein said other anti-HCV agent is ribavirin.

**14.** The pharmaceutical composition according to claim 11, wherein said other anti-HCV agent is selected from inhibitors of: helicase, NS2/3 protease and internal ribosome entry site (IRES).

**15.** The pharmaceutical composition according to claim 11, wherein said other anti-HCV agent is an inhibitor of HCV polymerase.

**16.** Use of a compound of formula I according to any one of claims 1 to 9 for the manufacture of a medicament for the treatment or prevention of hepatitis C viral infection.

**17.** Use of the composition according any one of claims 10 to 14 for the manufacture of a medicament for the treatment or prevention of a hepatitis C viral infection in a mammal .

**18.** Use a compound of formula I according to any one of claims 1 to 9, or a therapeutically acceptable salt thereof and one or more other anti-HCV agent for the manufacture of a medicament for the treatment or prevention of a hepatitis C viral infection in a mammal.

**19.** The use according to claim 18, wherein said other anti-HCV agent is selected from: α-interferon or pegylated α-interferon.

**20.** The use according to claim 18, wherein said other anti-HCV agent is ribavirin.

**21.** The use according to claim 18, wherein said other anti-HCV agent is selected from inhibitors of: helicase, NS2/3 protease and internal ribosome entry site (IRES).

**22.** The use according to claim 18, wherein said other anti-HCV agent is an inhibitor of HCV polymerase.

**Patentansprüche**

**1.** Verbindung der Formel I:

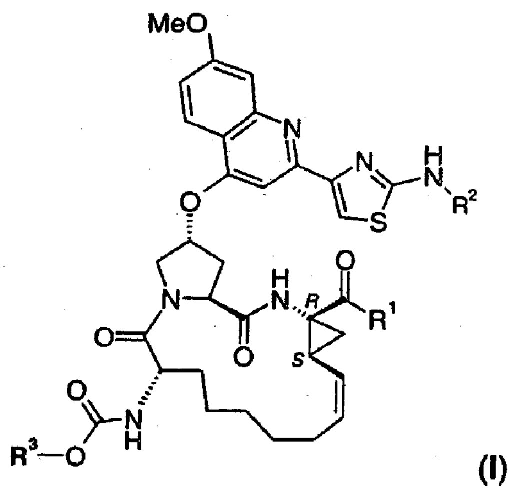

**(I)**

worin $\mathbf{R^1}$ Hydroxy oder $NHSO_2\mathbf{R^{1A}}$ darstellt, worin $\mathbf{R^{1A}}$ $(C_{1-8})$Alkyl, $(C_{3-7})$Cycloalkyl oder {$(C_{1-6})$Alkyl-$(C_{3-7})$cycloalkyl} ist, alle gegebenenfalls 1 bis 3 mal substituiert mit Halo, Cyano, Nitro, $O(C_{1-6})$Alkyl, Amido, Amino, oder Phenyl; oder $\mathbf{R^{1A}}$ ist $C_6$- oder $C_{10}$-Aryl, welches gegebenenfalls 1 bis 3 mal mit Halo, Cyano, Nitro, $(C_{1-6})$Alkyl, $O(C_{1-6})$Alkyl, Amido, Amino oder Phenyl substituiert ist; $\mathbf{R^2}$ ist $(C_{5-6})$-Cycloalkyl und $\mathbf{R^3}$ ist Cyclopentyl; oder ein pharmazeutisch akzeptables Salz hiervon.

**2.** Verbindung nach Anspruch 1, worin $\mathbf{R^1}$ Hydroxy oder $NHSO_2\mathbf{R^{1A}}$ darstellt, worin $\mathbf{R^{1A}}$ $(C_{1-6})$Alkyl, $(C_{3-7})$Cycloalkyl oder {$(C_{1-6})$Alkyl-$(C_{3-7})$cycloalkyl} ist, welche alle gegebenenfalls 1 bis 3 mal mit Halo, Nitro oder $O(C_{1-6})$Alkyl substituiert sind, oder Phenyl, welches gegebenenfalls 1 bis 3 mal mit Halo, Nitro, $(C_{1-6})$Alkyl oder $O(C_{1-6})$Alkyl substituiert ist.

**3.** Verbindung nach Anspruch 2, worin $\mathbf{R^1}$ $NHSO_2\mathbf{R^{1A}}$ darstellt, worin $\mathbf{R^{1A}}$ Methyl, Ethyl, Cyclopropyl, Cyclobutyl, Cyclopentyl, Cyclopropylmethyl, Cyclohexylethyl, $CCl_3$, $CF_3$, Phenyl, 2-Fluorphenyl oder 4-Methylphenyl darstellt.

**4.** Verbindung nach Anspruch 3, worin $\mathbf{R^{1A}}$ Cyclopropyl darstellt.

**5.** Verbindung nach Anspruch 1 oder 2, worin $\mathbf{R^1}$ Hydroxy darstellt.

**6.** Verbindung nach irgendeinem der Ansprüche 1 bis 5, worin $\mathbf{R^2}$ Cyclopentyl darstellt.

**7.** Verbindung nach Anspruch 1 mit der nachfolgenden Formel 101:

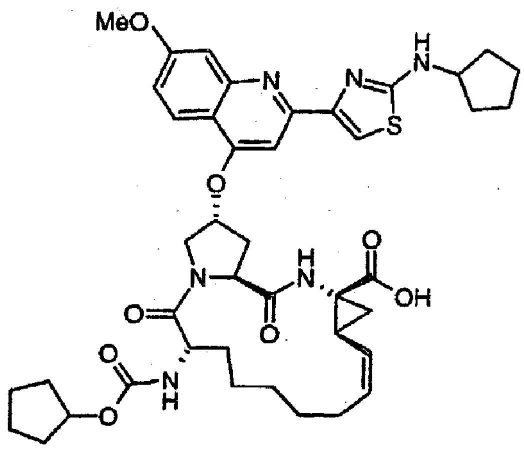

**(101).**

**8.** Verbindung nach Anspruch 1 mit der nachfolgenden Formel 102:

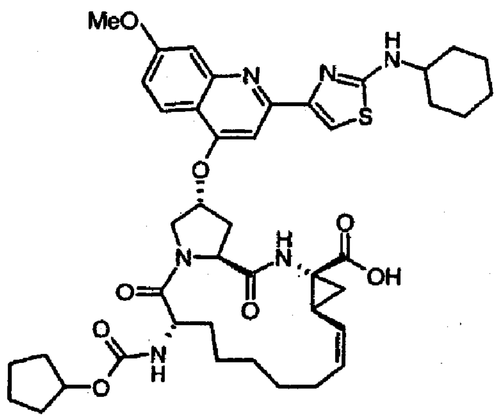

**(102).**

**9.** Verbindung nach Anspruch 1 mit der nachfolgenden Formel 103:

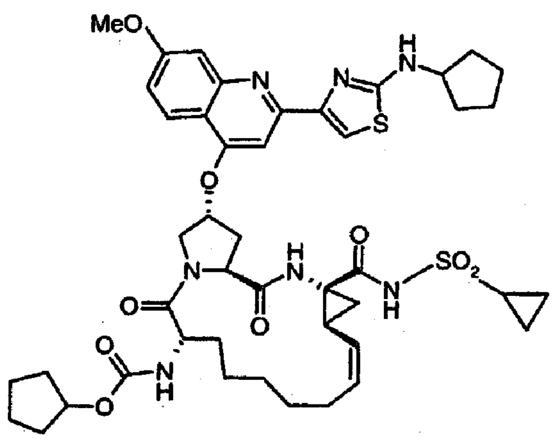

**(103).**

**10.** Pharmazeutische Zusammensetzung, umfassend eine gegen virale Hepatitis C wirksame Menge einer Verbindung der Formel I nach irgendeinem der Ansprüche 1 bis 9, oder ein pharmazeutisch akzeptables Salz hiervon, in Mischung mit einem pharmazeutisch akzeptablen Trägermedium oder Hilfsstoff.

**11.** Pharmazeutische Zusammensetzung nach Anspruch 10, weiterhin umfassend eine therapeutisch wirksame Menge von ein oder mehr Anti-HCV-Mitteln.

**12.** Pharmazeutische Zusammensetzung nach Anspruch 11, worin das andere Anti-HCV-Mittel ausgewählt ist aus: α-Interferon oder pegyliertem α-Interferon.

**13.** Pharmazeutische Zusammensetzung nach Anspruch 11, worin das andere Anti-HCV-Mittel Ribavirin darstellt.

**14.** Pharmazeutische Zusammensetzung nach Anspruch 11, worin das andere Anti-HCV-Mittel ausgewählt ist aus Inhibitoren von: Helicase, NS2/3-Protease und interner Ribosomeneintrittstelle (IRES).

**15.** Pharmazeutische Zusammensetzung nach Anspruch 11, worin das andere Anti-HCV-Mittel einen Inhibitor der HCV-Polymerase darstellt.

**16.** Verwendung einer Verbindung der Formel I nach irgendeinem der Ansprüche 1 bis 9 zur Herstellung eines Arzneimittels zur Behandlung oder Vorbeugung einer viralen Hepatitis C-Infektion.

**17.** Verwendung einer Zusammensetzung nach irgendeinem der Ansprüche 10 bis 14 zur Herstellung eines Arzneimittels zur Behandlung oder Vorbeugung einer viralen Hepatitis C-Infektion in einem Säuger.

**18.** Verwendung einer Verbindung der Formel I nach irgendeinem der Ansprüche 1 bis 9 oder eines therapeutisch akzeptablen Salzes hiervon und ein oder mehr Anti-HCV-Mitteln zur Herstellung eines Arzneimittels zur Behandlung oder Vorbeugung einer viralen Hepatitis C-Infektion in einem Säuger.

**19.** Verwendung nach Anspruch 18, wobei das andere Anti-HCV-Mittel ausgewählt ist aus: α-Interferon oder pegyliertem α-Interferon.

**20.** Verwendung nach Anspruch 18, wobei das andere Anti-HCV-Mittel Ribavirin darstellt.

**21.** Verwendung nach Anspruch 18, wobei das andere Anti-HCV-Mittel ausgewählt ist aus Inhibitoren von: Helicase, NS2/3-Protease und interner Ribosomeneintrittstelle (IRES).

**22.** Verwendung nach Anspruch 18, wobei das andere Anti-HCV-Mittel einen Inhibitor der HCV-Polymerase darstellt.

**Revendications**

**1.** Composé de formule I :

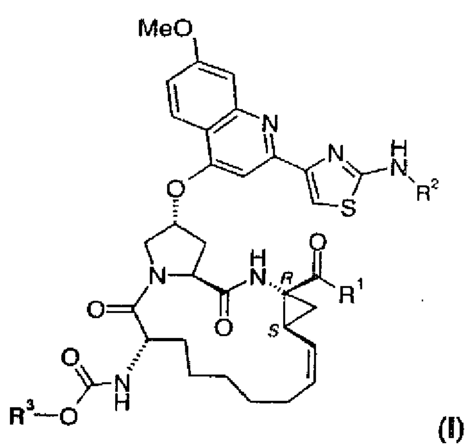

(I)

où $\mathbf{R^1}$ est hydroxy ou $NHSO_2\mathbf{R^{1A}}$ où $\mathbf{R^{1A}}$ est $(C_{1-8})$alkyle, $(C_{3-7})$cycloalkyle ou {$(C_{1-6})$alkyl-$(C_{3-7})$cycloalkyle}, qui sont tous éventuellement substitués de 1 à 3 fois avec halo, cyano, nitro, $O(C_{1-6})$alkyle, amido, amino ou phényle,

ou bien $R^{1A}$ est $C_6$ ou $C_{10}$ aryle qui est éventuellement substitué de 1 à 3 fois avec halo, cyano, nitro, $(C_{1-6})$alkyle, $O(C_{1-6})$alkyle, amido, amino ou phényle ; $R^2$ est $(C_{5-6})$cycloalkyle et $R^3$ est cyclopentyle ; ou sel pharmaceutiquement acceptable de celui-ci.

**2.** Composé selon la revendication 1 où $R^1$ est hydroxy ou $NHSO_2R^{1A}$ où $R^{1A}$ est $(C_{1-6})$alkyle, $(C_{3-7})$cycloalkyle ou $\{(C_{1-6})$alkyl-$(C_{3-7})$cycloalkyle$\}$, qui sont tous éventuellement substitués de 1 à 3 fois avec halo, nitro ou $O(C_{1-6})$ alkyle, ou phényle qui est éventuellement substitué de 1 à 3 fois avec halo, nitro, $(C_{1-6})$alkyle ou $O(C_{1-6})$alkyle.

**3.** Composé selon la revendication 2 où $R^1$ est $NHSO_2R^{1A}$ où $R^{1A}$ est méthyle, éthyle, cyclopropyle, cyclobutyle, cyclopentyle, cyclopropylméthyle, cyclohexyléthyle, $CCl_3$, $CF_3$, phényle, 2-fluorophényle ou 4-méthylphényle.

**4.** Composé selon la revendication 3 où $R^{1A}$ est cyclopropyle.

**5.** Composé selon la revendication 1 ou 2 où $R^1$ est hydroxy.

**6.** Composé selon l'une quelconque des revendications 1 à 5 où $R^2$ est cyclopentyle.

**7.** Composé selon la revendication 1 ayant la formule 101 suivante :

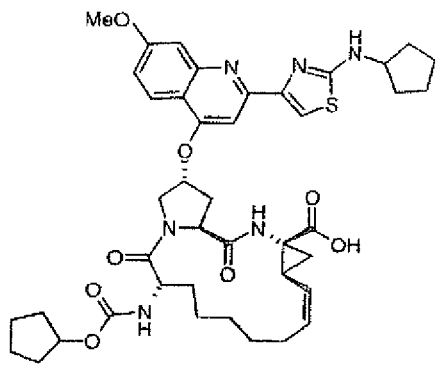

(101).

**8.** Composé selon la revendication 1 ayant la formule 102 suivante :

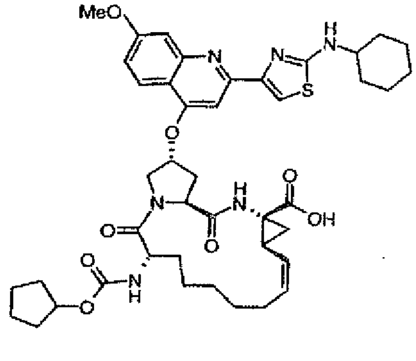

(102).

**9.** Composé selon la revendication 1 ayant la formule 103 suivante :

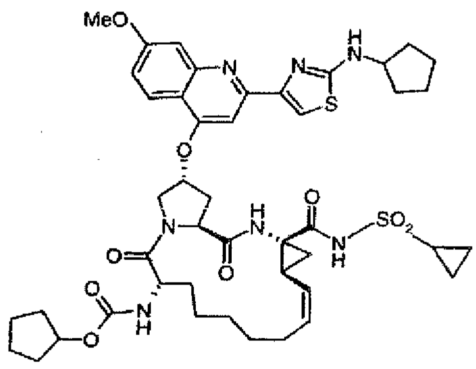

**(103).**

**10.** Composition pharmaceutique comprenant une quantité anti-virus de l'hépatite C efficace d'un composé de formule I selon l'une quelconque des revendications 1 à 9, ou d'un sel pharmaceutiquement acceptable de celui-ci, en mélange avec un milieu vecteur ou agent auxiliaire pharmaceutiquement acceptable.

**11.** Composition pharmaceutique selon la revendication 10 comprenant en outre une quantité thérapeutiquement efficace d'un ou plusieurs autres agents anti-HCV.

**12.** Composition pharmaceutique selon la revendication 11 où ledit autre agent anti-HCV est choisi parmi : l'interféron α et l'interféron α pégylé.

**13.** Composition pharmaceutique selon la revendication 11 où ledit autre agent anti-HCV est la ribavirine.

**14.** Composition pharmaceutique selon la revendication 11 où ledit autre agent anti-HCV est choisi parmi les inhibiteurs de : hélicase, protéase NS2/3 et site d'entrée interne des ribosomes (IRES).

**15.** Composition pharmaceutique selon la revendication 11 où ledit autre agent anti-HCV est un inhibiteur de polymérase de HCV.

**16.** Utilisation d'un composé de formule I selon l'une quelconque des revendications 1 à 9 pour la fabrication d'un médicament pour le traitement ou la prévention d'une infection à virus de l'hépatite C.

**17.** Utilisation de la composition selon l'une quelconque des revendications 10 à 14 pour la fabrication d'un médicament pour le traitement ou la prévention d'une infection à virus de l'hépatite C chez un mammifère.

**18.** Utilisation d'un composé de formule I selon l'une quelconque des revendications 1 à 9, ou d'un sel thérapeutiquement acceptable de celui-ci et d'un ou plusieurs autres agents anti-HCV pour la fabrication d'un médicament pour le traitement ou la prévention d'une infection à virus de l'hépatite C chez un mammifère.

**19.** Utilisation selon la revendication 18 où ledit autre agent anti-HCV est choisi parmi : l'interféron α et l'interféron α pégylé.

**20.** Utilisation selon la revendication 18 où ledit autre agent anti-HCV est la ribavirine.

**21.** Utilisation selon la revendication 18 où ledit autre agent anti-HCV est choisi parmi les inhibiteurs de : hélicase, protéase NS2/3 et site d'entrée interne des ribosomes (IRES).

**22.** Utilisation selon la revendication 18 où ledit autre agent anti-HCV est un inhibiteur de polymérase de HCV.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 2337262 A
- JP 10298151 B
- JP 11126861 B
- JP 11292840 B
- JP 2001103993 A
- US 6159938 A
- US 6187905 B
- WO 9743310 A
- WO 9817679 A
- WO 9822496 A
- WO 9846597 A
- WO 9846630 A
- WO 9938888 A
- WO 9950230 A
- WO 9964442 A
- WO 9907733 A
- WO 9907734 A
- WO 0009543 A
- WO 0009558 A
- WO 0020400 A
- WO 0059929 A
- WO 0031129 A
- WO 0102424 A
- WO 0107407 A
- WO 0116357 A
- WO 0132691 A
- WO 0140262 A
- WO 0158929 A
- WO 0164678 A
- WO 0174768 A
- WO 0177113 A
- WO 0181325 A
- WO 0208187 A
- WO 0208198 A
- WO 0208244 A
- WO 0208251 A
- WO 0208256 A
- WO 0218369 A
- WO 0260926 A
- WO 0279234 A
- WO 02060926 A
- US 10198680 B
- CA 0201127 W
- US 10198384 A
- CA 0201128 W
- US 10198259 B
- CA 0201129 W
- WO 02100846 A1
- WO 02100851 A2
- WO 0185172 A1
- WO 02098424 A1
- WO 0006529 A
- WO 0206246 A1
- WO 0147883 A
- WO 03000254 A
- EP 1256628 A2
- WO 0190121 A2
- WO 02069903 A2
- WO 02057287 A2
- WO 02057425 A2
- WO 02052015 A
- US 2369711 A


### Non-patent literature cited in the description


- **BERGER A. ; SCHECHTER I.** *Transactions of the Royal Society London,* 1970, vol. B257, 249-264
- **S.M. BIRGE et al.** *J. Pharm. Sci.,* 1977, vol. 66, 1-19
- Remington's Pharmaceutical Sciences. The Science and Practice of Pharmacy. Mack Publishing Company, 1995
- **MITSUNOBU.** *Synthesis,* January 1981, 1-28
- **RANO et al.** *Tet. Lett.,* 1994, vol. 36, 3779-3792
- **KRCHNAK et al.** *Tet. Lett.,* 1995, vol. 36, 6193-6196
- **MILLER, S.J. ; BLACKWELL, H.E. ; GRUBBS, R.H.** *J. Am. Chem. Soc.,* 1996, vol. 118, 9606-9614
- **KINGSBURY, J.S. ; HARRITY, J.P.A. ; BONITATEBUS, P.J. ; HOVEYDA, A.H.** *J. Am. Chem. Soc.,* 1999, vol. 121, 791-799
- **HUANG, J. ; STEVENS, E.D. ; NOLAN, S.P. ; PETERSEN, J.L.** *J. Am. Chem. Soc.,* 1999, vol. 121, 2674-2678

- **W.C. STILL et al.** *J. Org. Chem.,* 1978, vol. 43, 2923
- **LOHMAN et al.** *Science,* 1999, vol. 285, 110-113
- **MARTELL et al.** *J. Clin. Microbiol.,* 1999, vol. 37, 327-332